# EUROPEAN PATENT SPECIFICATION

(11) **EP 2 114 130 B1**
(45) Date of publication and mention of the grant of the patent: **20.07.2011**
(21) Application number: 08701838.8
(22) Date of filing: 17.01.2008
(51) Int. Cl.: A01K 67/027, A61K 48/00, A61K 38/17, A61P 25/30, A61P 25/20, G01N 33/50, C12Q 1/68

(54) **ANIMAL MODEL FOR ADHD**
TIERMODELL FÜR ADHD
MODELE ANIMAL DE L'ADHD

(30) Priority: 17.01.2007 GB 0700894
(43) Date of publication of application: 11.11.2009
(73) Proprietor: UCL Business PLC, London W1T 4TP (GB)
(72) Inventor: STANFORD, Susan Clare, London N6 5HP (GB); GURLING, Hugh, London SE21 7DH (GB); HUNT, Steven, London WClE 6BT (GB)
(74) Representative: Wallis, Naomi Rachel
(86) International application number: PCT/GB2008/000160
(87) International publication number: WO 2008/087419

(56) References cited:
- HERPFER I ET AL: "A comparison of neurokinin 1 receptor knock-out (NK1-/-) and wildtype mice: exploratory behaviour and extracellular noradrenaline concentration in the cerebral cortex of anaesthetised subjects" NEUROPHARMACOLOGY, PERGAMON PRESS, OXFORD, GB, vol. 48, no. 5, April 2005 (2005-04), pages 706-719, XP004827321 ISSN: 0028-3908
- DAVIDS E ET AL: "Animal model of attention-deficit hyperactivity disorder" BRAIN RESEARCH REVIEWS, ELSEVIER, XX, vol. 42, April 2003 (2003-04), pages 1-21, XP002983717 ISSN: 0165-0173
- FLIERS E.A. ET AL: "Undertreatment of motor problems in children with ADHD", CHILD ADOLESC. MENT. HEALTH, vol. 15, no. 2, 21 August 2009 (2009-08-21), pages 85-90,
- JAMES A., LAI F.H. & DAHL C.: "Attention deficit hyperactivity disorder and suicide: a review of possible associations.", ACTA PSYCHIATR. SCAND., vol. 110, 2004, pages 408-415,

## Description

The invention relates to an animal model for attention deficit hyperactivity disorder, methods for making such models and methods for using such models.

Attention deficit hyperactivity disorder (ADHD) affects around 5% of children in the UK and USA. Symptoms of ADHD include hyperactivity, inattentiveness, impulsivity and clumsiness. ADHD is strongly heritable and has close links to drug, especially alcohol dependence. Previous studies have led to the conclusion that ADHD is associated with an abnormal monoaminergic transmission, especially of dopamine and noradrenaline in key brain circuits. The calming effect of psychostimulants such as d-amphetamine (d-AMP) and methylphenidate in subjects with ADHD supports this view.

ADHD is not especially well understood and it is still difficult to diagnose. Several putative animal models for ADHD have been developed but none expresses all its core features (Davids, 2003, Brain Res. Reviews 42, 1-21). It would be very advantageous to have an animal model to aid further research into the condition and potential treatments thereof.

Additionally, it would be useful to identify a marker for a predisposition to ADHD and related conditions that could be used to identify subjects more likely to suffer from ADHD and related conditions and to help confirm diagnosis thereof.

Mice lacking functioning substance P preferring receptors (NK1^{-/-}) have been shown to have an increased central noradrenergic transmission and abnormalities in their behaviour when compared with their NK1^{+/+} counterparts (Herpfer, 2005, Neuropharmacology 48, 706-719). The inventors have now surprisingly found that these mice also have

impaired dopamine and noradrenaline transmission, hyperactivity, impulsivity and inattentiveness, paradoxical behaviour in response to d-AMP and methylphenidate and other signs of ADHD.

Hence, according to the invention, there is provided the use of an NK1^{-/-} rodent as an animal model for ADHD. Related diseases can also be studied with this model: alcohol addiction, dyspraxia, conduct disorder, self harm or suicidality. Also provided is the use of a rodent having a functional substance P receptor to which an antagonist of the substance P receptor has been administered as an animal model for ADHD. Related diseases can also be studied with this model: alcohol addiction, dyspraxia, conduct disorder, self harm or suicidality.

NK1^{-/-} animals are well known in the art. The term NK1^{-/-} animal is used herein to mean any animal that lacks functional substance P receptors or which has a significantly reduced number of substance P receptors when compared with a wild-type animal. The animal may lack the receptors or have a reduced number of receptors for any reason, such as lacking a gene encoding the NK1 receptor, or having a gene encoding a nonfunctional NK1 receptor. It includes naturally occurring animals and animals that have been bred or altered to lack the receptors. One skilled in the art could produce such animals.

One procedure for targeted disruption of the NK1 receptor gene is described in *de Felipe et al., (1998)* and was based on that described in *Nehls et al. (1996).* Briefly, homologous recombination in embryonic stem cells may be used to create a mouse line in which the NK-1 receptor gene is disrupted in exon 1. For disruption the inventors used a cassette containing the β-galactosidase coding region preceded by internal ribosomal entry sequence (IRES), followed by the neomycin coding region.

Any other method of disrupting the NK1 receptor gene or reducing NK1 gene product expression may be used. Such methods include using antisense nucleic acids, triple helix methods and any other well known methods.

Although in the prior art, NK1^{-/-} animals have been shown to display some characteristics associated with ADHD, it has not previously been shown that such mice can display all the core characteristics, nor have such animals been considered as models for ADHD or other conditions. (Herpfer, 2005)

As indicated above, ADHD is attention deficit hyperactivity disorder. Included in the definition of ADHD used herein is ADD, attention deficit disorder. ADHD is a common developmental and behavioral disorder, seen in children, but which can remain into adulthood. It is characterized by poor concentration, distractibility, hyperactivity, and impulsiveness that are inappropriate for the child's age. Children and adults with ADHD are easily distracted by sights and sounds in their environment, cannot concentrate for long periods of time, are restless and impulsive, or have a tendency to daydream and be slow to complete tasks. The core characteristics of ADHD are described in Table 1.In the animal model of the invention, the rodent shows reduced locomotor activity in response to methylphenidate or d-amphetamine

It has not previously been shown that the characteristics of NK1^{-/-} animals can be reproduced in animals that are wild-type for the NK1 gene (NK1^{+/+}) or which display normal substance P receptor function by administering an antagonist to the substance P receptor. Antagonists for the substance P receptor include, but are not limited to , CGP 49823, CP .122721, CP 99994, CP 96345, FK 224, FK 888, GR 597599, GR 82334, GR 203040, GR 205171, GR 679769, GW 823296, GW 597599, L303870, L703606, L733060, L 668169, L 732138, L 754030 / MK869, L 760735, LY 686017, MDL 103392, MK 869 / L 754030, MPC 4505, NKP 608, R 116301, RP 67580, RPR 100893, SDZ NKT 343, SR 140333, Netupitant, and Befetupitant. All the above antagonists are well known in the art and are commercially available or may be synthesised by one skilled in the art.

The substance P receptor, also called the tachykinin 1 receptor (TACR1) or NK1 receptor is well known in the art; however, its role in monoamine transmission is not fully understood. The inventors believe the substance P receptor to be impaired in subjects with ADHD, explaining the changes displayed in monoamine transmission in ADHD subjects.

The animal is a rodent, especially a rat, gerbil, guinea pig or a mouse. The animal is most preferably a mouse.

An animal model, as is known in the art, is a non-human animal having or displaying the characteristics of a disease or condition. The use as an animal model means any use of an animal to study the disease or condition, such as the use to study the progression or development or the response to new or existing treatments.

Alcoholism or alcohol addiction, as used herein, means a dependence on alcohol, characterized by repeated excessive use of alcoholic beverages and the development of withdrawal symptoms on reducing or ceasing alcohol intake.

Conduct disorder is a type of disruptive behavior disorder generally seen in childhood and adolescence involving a persistent pattern of behaviour in which the rights of others or the norms or rules of society are violated, with misconduct including aggression to people or animals, destruction of property, deceitfulness or theft, and serious violations of rules.

Dyspraxia is considered to be an impairment or immaturity of the organisation of movement, including problems of language, perception and thought. Other names for dyspraxia include Clumsy Child Syndrome, Developmental Co-ordination Disorder, Minimal Brain Dysfunction, Motor learning Difficulty, and Perceptuo-motor Dysfunction.

Self harm or injury is when someone deliberately hurts or injures him or herself. This can take a number of forms including, but not limited to, cutting, taking overdoses of tablets or medicines, punching, scratching, picking or tearing at one's skin causing sores and scarring, burning and inhaling or sniffing harmful substances.

Suicidality means suicidal thinking or behaviour and includes considering or attempting self-destructive actions.

Also anticipated, though not disclosed in detail, is the use of a cell, tissue or organ from a NK1^{-/-} animal or a NK1^{-/-} cell or a tissue or organ made up of such cells in a cellular model for ADHD. Such cellular models include any sample which contains cells or genetic material, such as blood, urine, brain and cerebrospinal fluid.

The disclosure also provides for reference purposes a transgenic animal having altered NK1 gene expression. Animals of any species, including, but not limited to, mice, rats, rabbits, guinea pigs, pigs, micro-pigs, goats, sheep, and non-human primates, e.g., baboons, monkeys, and chimpanzees may be used to generate NK1 gene transgenic animals. Such animals may express an NK1 gene sequence from a different species (e.g., mice expressing human NK1 gene sequences), or may have been genetically engineered to over express endogenous NK1 gene sequences.

Any technique known in the art may be used to introduce a NK1 gene transgene into animals to produce the founder lines of transgenic animals. Such techniques include, but are not limited to pronuclear microinjection (Hoppe and Wagner, 1989, U.S. Pat. No. 4,873,191); retrovirus mediated gene transfer into germ lines (Van der Putten, et al., 1985, Proc. Natl. Acad. Sci., USA 82, 6148-6152); gene targeting in embryonic stem cells (Thompson, et al., 1989, Cell 56, 313-321); electroporation of embryos (Lo, 1983, Mol. Cell. Biol. 3,1803-1814); and sperm-mediated gene transfer (Lavitrano et al., 1989, Cell 57, 717-723) (For a review of such techniques, see Gordon, 1989, Transgenic Animals, Intl. Rev. Cytol.115; 171-229).

Any technique known in the art may be used to produce transgenic animal clones containing a NK1 gene transgene; for example, nuclear transfer into enucleated oocytes of nuclei from cultured embryonic, fetal or adult cells induced to quiescence (Campbell, et al., 1996, Nature 380, 64-66; Wilmut, et al., Nature 385, 810-813).

In this context, the present disclosure provides for transgenic animals that carry a NK1 gene transgene in all their cells, as well as animals that carry the transgene in some, but not all their cells, i.e., mosaic animals. The transgene may be integrated as a single transgene or in concatamers, e.g., head-to-head tandems or head-to-tail tandems. The transgene may also be selectively introduced into and activated in a particular cell type by following, for example, the teaching of Lasko *et al.* (Lasko, et al., 1992, Proc. Natl. Acad. Sci. USA 89, 6232-6236). The regulatory sequences required for such a cell-type specific activation will depend upon the particular cell type of interest, and will be apparent to those of skill in the art. When it is desired that the NK1 gene transgene be integrated into the chromosomal site of the endogenous NK1 gene, gene targeting is preferred. Briefly, when such a technique is to be utilized, vectors containing some nucleotide sequences homologous to the endogenous NK1 gene are designed for the purpose of integrating, via homologous recombination with chromosomal sequences, into and disrupting the function of the nucleotide sequence of the endogenous NK1 gene. The transgene may also be selectively introduced into a particular cell type, thus inactivating the endogenous NK1 gene in only that cell type, by following, for example, the teaching of Gu, *et al.* (Gu, et al., 1994, Science 265, 103-106). The regulatory sequences required for such a cell-type specific inactivation will depend upon the particular cell type of interest, and will be apparent to those of skill in the art.

Once transgenic animals have been generated, the expression of the recombinant NK1 gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques that include but are not limited to Northern blot analysis of tissue samples obtained from the animal, in situ hybridization analysis, and RT-PCR (reverse transcriptase PCR). Samples of NK1 gene-expressing tissue may also be evaluated immunocytochemically using antibodies specific for the NK1 gene transgene product.

The invention also provides a method for identifying a compound that affects ADHD and possibly the related diseases alcoholism, dyspraxia, conduct disorder, deliberate self harm or injury or suicidality comprising administering a test compound to a NK1^{-/-} animal or an animal treated with an antagonist to the substance P receptor and determining the effects on the behaviour of the animal.

The method of the invention is preferably for identifying a compound that reduces the symptoms or signs of such conditions.

A compound that reduces such symptoms or signs will lead to a change in the rodent's locomotor activity in response to methylphenidate or d-amphetamine. Also, it may preferably have at least one of the following effects on the rodent model: reduction of locomotor activity, especially in a stressful (e.g. aversive novel) environment such as a light/dark exploration box or open field; increased latency to return to a novel arena from a familiar zone; longer or less frequent visits to a novel area; improved motor co-ordination and/or learning; reduced impulsivity and further increase of locomotor activity in response to d-AMP.

A method in alternative to that of the invention could comprise administering a test compound to a NK1^{-/-}animal or an animal treated with an antagonist to the substance P receptor and determining changes in neurotransmitter function such as a reduction in noradrenaline release in the frontal cortex or an increase in extracellular dopamine in the frontal cortex. Monitoring behaviour will also exhibit changes and could be used in the screen.

Also, the method may be used to test for side effects of a compound that affects ADHD, alcoholism, dyspraxia, conduct disorder, deliberate self harm or injury or suicidality. For instance, therapies that activate NK1 receptors might cause nausea. This may be tested for using the Conditioned Taste Avoidance test, in which a test compound is either flavoured or combined with an inactive, flavoured substance. If the test compound causes nausea, the animal avoids that flavour in future.

With regard to intervention, any treatments that reverse any aspect of symptoms of a NK1 gene disorder should be considered as candidates for human therapeutic intervention in such a disorder. Dosages of test agents may be determined by, for example, deriving dose-response curves.

Also disclosed for reference purposes is an isolated protein encoded by the sequence of an NK1 gene containing at least one polymorphism or which encodes a non-functional NK1 receptor.

Also disclosed for reference purposes is an isolated RNA molecule that is complementary to the DNA sequence of an NK1 gene containing at least one polymorphism or which encodes a non-functional NK1 receptor.

Isolated proteins and RNA molecules as mentioned above are known herein as gene products.

Additionally disclosed are antibodies that bind to the isolated protein or RNA molecule. It is preferred that the antibodies do not bind to the wild-type NK1 protein, or to RNA that is complementary to the wild-type NK1 gene. The term antibody is intended to mean a whole antibody or a functional fragment thereof, i.e. any part of an antibody that is able to bind to the protein. Such antibodies may include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

Such antibodies may be useful in the diagnosis or treatment of ADHD, alcoholism, dyspraxia, conduct disorder, deliberate self harm or injury or suicidality. Such antibodies may be used, for example, in the detection of a NK1 gene product in a biological sample and may, therefore, be utilized as part of a diagnostic or prognostic technique whereby patients may be tested for the presence of abnormal forms of NK1 gene products. Such antibodies may also be utilized in conjunction with, for example, compound screening schemes for the evaluation of the effect of test compounds on NK1 gene product levels and/or activity.

Also anticipated, but not disclosed in all their details, are methods of identifying candidate compounds for use in the treatment of ADHD, alcohol addiction, dyspraxia, conduct disorder, self harm or suicidality comprising identifying compounds that bind to a NK1 gene product, intracellular proteins or portions of proteins that interact with a NK1 gene product, compounds that interfere with the interaction of a NK1 gene product with intracellular proteins and compounds that modulate the activity of NK1 gene (i.e., modulate the level of NK1 gene expression and/or modulate the level of NK1 gene product activity). Assays may additionally be utilized that identify compounds that bind to NK1 gene regulatory sequences (e.g.,promoter sequences; see e.g., Platt, 1994, J. Biol. Chem. 269, 28558-28562), and that may modulate the level of NK1 gene expression. Additionally, once identified, the effect of a compound on any one or more of the conditions may be tested, by administering the compound to a subject having a condition or to an animal model and observing the effects on the condition. Compounds may include, but are not limited to, small organic molecules, such as ones that are able to cross the blood-brain barrier, gain entry into an appropriate cell and affect expression of the NK1 gene or some other gene involved in a NK1 regulatory pathway, or intracellular proteins.

Compounds may include, but are not limited to, peptides such as, for example, soluble peptides, including but not limited to, Ig-tailed fusion peptides, and members of random peptide libraries; (see, e.g., Lam, et al., 1991, Nature 354, 82-84; Houghten, et al., 1991, Nature 354, 84-86), and combinatorial chemistry-derived molecular library made of D- and/or L- configuration amino acids, phosphopeptides (including, but not limited to members of random or partially degenerate, directed phosphopeptide libraries; see, e.g., Songyang, et al., 1993, Cell 72, 767-778), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), and small organic or inorganic molecules. Such compounds may further comprise compounds, in particular drugs or members of classes or families of drugs, known to ameliorate or exacerbate the symptoms of a neuropsychiatric disorder such as conduct disorder, dis-social personality disorder, attention deficit hyperactivity disorder and alcoholism. Such compounds include amphetamine, methylphenidate, d-amphetamine, modafanil, and other stimulants, guanfacine and antidepressants, such as fluoxetine and imipramine, and atomoxetine.

Compounds identified via assays such as those described herein may be useful, for example, in elaborating the biological function of the NK1 gene product, and for ameliorating NK1 gene disorders or neuropsychiatric disorders, such as conduct disorder, attention deficit hyperactivity disorder and alcoholism. Assays for testing the effectiveness of compounds are discussed below.

The principle of the assays used to identify compounds that bind to the NK1 gene product involves preparing a reaction mixture of the NK1 gene product and the test compound under conditions and for a time sufficient to allow the two components to interact and bind, thus forming a complex that can be removed and/or detected in the reaction mixture. These assays can be conducted in a variety of ways. For example, one method to conduct such an assay would involve anchoring NK1 gene product or the test substance onto a solid phase and detecting NK1 gene product/test compound complexes anchored on the solid phase at the end of the reaction. In one embodiment of such a method, the NK1 gene product may be anchored onto a solid surface, and the test compound, which is not anchored, may be labeled, either directly or indirectly.

In practice, microtiter plates may conveniently be utilized as the solid phase. The anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labelled antibody specific for the previously non-immobilized component (the antibody, in turn, may be directly labeled or indirectly labeled with a labeled anti-Ig antibody).

Alternatively, a reaction can be conducted in a liquid phase, the reaction products separated from unreacted components, and complexes detected; e.g., using an immobilized antibody specific for NK1 gene product or the test compound to anchor any complexes formed in solution, and a labeled antibody specific for the other component of the possible complex to detect anchored complexes.

Any method suitable for detecting protein-protein interactions may be employed for identifying NK1 gene protein-protein interactions.

Among the traditional methods that may be employed are co-immunoprecipitation, cross-linking and co-purification through gradients or chromatographic columns. Utilizing procedures such as these allows for the identification of proteins, including intracellular proteins, that interact with NK1 gene products. Once isolated, such a protein can be identified and can be used, in conjunction with standard techniques, to identify proteins it interacts with. For example, at least a portion of the amino acid sequence of a protein that interacts with the NK1 gene product can be ascertained using techniques well known to those of skill in the art, such as via the Edman degradation technique (see, e.g., Creighton, 1983, "Proteins: Structures and Molecular Principles," W.H. Freeman & Co., N.Y., pp.34-49). The amino acid sequence obtained may be used as a guide for the generation of oligonucleotide mixtures that can be used to screen for gene sequences encoding such proteins. Screening may be accomplished, for example, by standard hybridization or PCR techniques. Techniques for the generation of oligonucleotide mixtures and the screening are well-known. (See, e.g., Ausubel, supra, and 1990, "PCR Protocols: A Guide to Methods and Applications," Innis, et al., eds. Academic Press, Inc., New York).

Additionally, methods may be employed that result in the simultaneous identification of genes that encode a protein which interacts with an NK1 gene protein. These methods include, for example, probing expression libraries with labeled NK1 gene protein, using NK1 gene protein in a manner similar to the well known technique of antibody probing of lambda.gt10 and lambda.gt10 libraries.

One method that detects protein interactions in vivo, the two-hybrid system, is described in detail for illustration only and not by way of limitation. One version of this system has been described (Chien, et al., 1991, Proc. Natl. Acad. Sci. USA, 88, 9578-9582) and is commercially available from Clontech (Palo Alto, Calif.).

Briefly, utilizing such a system, plasmids are constructed that encode two hybrid proteins: one consists of the DNA-binding domain of a transcription activator protein fused to the NK1 gene product and the other consists of the transcription activator protein's activation domain fused to an unknown protein that is encoded by a cDNA that has been recombined into this plasmid as part of a cDNA library. The DNA-binding domain fusion plasmid and the cDNA library are transformed into a strain of the yeast Saccharomyces cerevisiae that contains a reporter gene (e.g., HBS or lacZ) whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, NK1 gene products may be used as the bait gene product. Total genomic or cDNA sequences are fused to the DNA encoding an activation domain. This library and a plasmid encoding a hybrid of a bait NK1 gene product fused to the DNA-binding domain are co-transformed into a yeast reporter strain, and the resulting transformants are screened for those that express the reporter gene. For example, and not by way of limitation, a bait NK1 gene sequence, such as the open reading frame of the NK1 gene, can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait NK1 gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait NK1 gene-GAL4 fusion plasmid into a yeast strain that contains a lacZ gene driven by a promoter that contains GAL4 activation sequence. A cDNA encoded protein, fused to GAL4 transcriptional activation domain, that interacts with bait NK1 gene product will reconstitute an active GAL4 protein and thereby drive expression of the HIS3 gene. Colonies that express HIS3 can be detected by their growth on Petri dishes containing semi-solid agar based media lacking histidine. The cDNA can then be purified from these strains, and used to produce and isolate the bait NK1 gene-interacting protein using techniques routinely practiced in the art.

NK1 gene products of the invention may, in vivo, interact with one or more macromolecules, including intracellular macromolecules, such as proteins. Such macromolecules may include, but are not limited to, nucleic acid molecules and those proteins identified via methods known in the art. For purposes of this discussion, the macromolecules are referred to herein as "binding partners". Compounds that disrupt NK1 gene binding in this way may be useful in regulating the activity of the NK1 gene product, especially mutant NK1 gene products.

The basic principle of the assay systems used to identify compounds that interfere with the interaction between the NK1 gene product and its binding partner or partners involves preparing a reaction mixture containing the NK1 gene product, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of NK1 gene product and its binding partner. Control reaction mixtures are incubated without the test compound or with a placebo. The formation of any complexes between the NK1 gene protein and the binding partner is then detected. The formation of a complex in the control reaction, but not in the reaction mixture containing the test compound, indicates that the compound interferes with the interaction of the NK1 gene protein and the interactive binding partner. Additionally, complex formation within reaction mixtures containing the test compound and normal NK1 gene protein may also be compared to complex formation within reaction mixtures containing the test compound and a mutant NK1 gene protein. This comparison may be important in those cases wherein it is desirable to identify compounds that disrupt interactions of mutant but not normal NK1 gene proteins.

The assay for compounds that interfere with the interaction of the NK1 gene products and binding partners can be conducted in a heterogeneous or homogeneous format. Heterogeneous assays involve anchoring either the NK1 gene product or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the NK1 gene products and the binding partners, e.g., by competition, can be identified by conducting the reaction in the presence of the test substance; i.e., by adding the test substance to the reaction mixture prior to or simultaneously with the NK1 gene protein and interactive intracellular binding partner. Alternatively, test compounds that disrupt preformed complexes, e.g., compounds with higher binding constants that displace one of the components from the complex, can be tested by adding the test compound to the reaction mixture after complexes have been formed. The various formats are described briefly below.

In a heterogeneous assay system, either the NK1 gene product or the interactive binding partner, is anchored onto a solid surface, while the non-anchored species is labeled, either directly or indirectly. In practice, microtitre plates are conveniently utilized. The anchored species may be immobilized by non-covalent or covalent attachments. Non-covalent attachment may be accomplished simply by coating the solid surface with a solution of the NK1 gene product or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (e.g., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labelled, an indirect label can be used to detect complexes anchored on the surface; e.g., using a labeled antibody specific for the initially non-immobilized species (the antibody, in turn, may be directly labeled or indirectly labeled with a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex formation or that disrupt preformed complexes can be identified.

In an alternate embodiment of the invention, a homogeneous assay can be used. In this approach, a preformed complex of the NK1 gene protein and the interactive binding partner is prepared in which either the NK1 gene product or its binding partners is labeled, but the signal generated by the label is quenched due to complex formation (see, e.g., U.S. Pat. No. 4,109,496 by Rubenstein which utilizes this approach for immunoassays). The addition of a test substance that competes with and displaces one of the species from the preformed complex will result in the generation of a signal above background. In this way, test substances that disrupt NK1 gene protein/binding partner interaction can be identified.

The NK1 gene product can be prepared for immobilization using standard recombinant DNA techniques. For example, the NK1 gene coding region can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art.

This antibody can be labeled with the radioactive isotope ¹²⁵I, for example, by methods routinely practiced in the art. In a heterogeneous assay, e.g., the GST-NK1 gene fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between the NK1 gene protein and the interactive binding partner can be detected by measuring the amount of radioactivity that remains associated with the glutathione-agarose beads. A successful inhibition of the interaction by the test compound will result in a decrease in measured radioactivity.

Alternatively, the GST-NK1 gene fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the NK1 gene product/binding partner interaction can be detected by adding the labeled antibody and measuring the radioactivity associated with the beads.

These same techniques can be employed using peptide fragments that correspond to the binding domains of the NK1 gene protein and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensating mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a short, labelled peptide comprising the binding domain may remain associated with the solid material, which can be isolated and identified by amino acid sequencing. Also, once the gene coding for the segments can be engineered to express peptide fragments of the protein, which can then be tested for binding activity and purified or synthesized.

For example, and not by way of limitation, a NK1 gene product can be anchored to a solid material by making a GST-NK1 gene fusion protein and allowing it to bind to glutathione agarose beads. The interactive binding partner obtained can be labeled with a radioactive isotope, such as ³⁵S, and cleaved with a proteolytic enzyme such as trypsin. Cleavage products can then be added to the anchored GST-NK1 gene fusion protein and allowed to bind. After washing away unbound peptides, labelled bound material, representing the binding partner binding domain, can be eluted, purified, and analyzed for amino acid sequence by well-known methods. Peptides so identified can be produced synthetically or fused to appropriate facilitative proteins using recombinant DNA technology.

Compounds, including but not limited to binding compounds identified via assay techniques such as those described can be tested for the ability to ameliorate symptoms of a NK1 gene disorder, including conduct disorder, attention deficit hyperactivity disorder and alcoholism. It should be noted that the assays described herein can identify compounds that affect NK1 gene activity by either affecting NK1 gene expression or by affecting the level of NK1 gene product activity. For example, compounds may be identified that are involved in another step in the pathway in which the NK1 gene and/or NK1 gene product is involved and, by affecting this same pathway, may modulate the effect of NK1 gene on the development of a neuropsychiatric disorder such as conduct disorder, attention deficit hyperactivity disorder and alcoholism. Such compounds can be used as part of a therapeutic method for the treatment of the disorder.

Also, cell-based systems can be used to identify compounds that may act to ameliorate symptoms of a NK1 gene disorder or a neuropsychiatric disorder, such as conduct disorder, attention deficit hyperactivity disorder and alcoholism. Such cell systems can include, for example, recombinant or non-recombinant cells, such as cell lines, that express the NK1 gene.

In utilizing such cell systems, cells that express NK1 gene may be exposed to a compound suspected of exhibiting an ability to ameliorate symptoms of a NK1 gene disorder or a neuropsychiatric disorder, such as conduct disorder, attention deficit hyperactivity disorder and alcoholism, at a sufficient concentration and for a sufficient time to be effective. After exposure, the cells can be assayed to measure alterations in the expression of the NK1 gene, e.g., by assaying cell lysates for NK1 gene mRNA transcripts (e.g., by Northern analysis) or for NK1 gene products expressed by the cell; compounds that modulate expression of the NK1 gene are good candidates as therapeutics. Alternatively, the cells are examined to determine whether one or more cellular phenotypes associated with an NK1 gene disorder or a neuropsychiatric disorder, such as conduct disorder, attention deficit hyperactivity disorder and alcoholism, has been altered to resemble a more normal or unimpaired, unaffected phenotype, or a phenotype more likely to produce a lower incidence or severity of disorder symptoms.

Additionally, such antibodies can be used in conjunction with the gene therapy techniques to, for example, evaluate the normal and/or engineered NK1 gene-expressing cells prior to their introduction into the patient.

Anti-NK1 gene product antibodies may additionally be used as a method for the inhibition of abnormal NK1 gene product activity. Thus, such antibodies may, therefore, be utilized as part of treatment methods for an NK1 gene disorder or a neuropsychiatric disorder, such as conduct disorder, attention deficit hyperactivity disorder and alcoholism. Accordingly, there is provided the use of an antibody to a protein encoded by or an RNA molecule complementary to an NK1 gene encoding a nonfunctional NK1 receptor in the preparation of a medicament for the treatment of ADHD, dyspraxia, alcoholism, conduct disorder, self harm or injury or suicidality.

For the production of antibodies against a NK1 gene product, various host animals may be immunized by injection with a NK1 gene product, or a portion thereof. Such host animals may include, but are not limited to rabbits, mice, and rats. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

Polyclonal antibodies are heterogeneous populations of antibody molecules derived from the sera of animals immunized with an antigen, such as a NK1 gene product, or an antigenic functional derivative thereof. For the production of polyclonal antibodies, host animals such as those described above, may be immunized by injection with NK1 gene product supplemented with adjuvants as also described above.

Monoclonal antibodies, which are homogeneous populations of antibodies to a particular antigen, may be obtained by any technique that provides for the production of antibody molecules by continuous cell lines in culture. These include, but are not limited to, the hybridoma technique of Kohler and Milstein, (1975, Nature 256, 495-497; and U.S. Pat. No. 4,376,110), the human B-cell hybridoma technique (Kosbor et al., 1983, Immunology Today 4, 72; Cole et al., 1983, Proc. Natl. Acad. Sci. USA 80, 2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison, et al., 1984, Proc. Natl. Acad. Sci., 81, 6851-6855; Neuberger, et al., 1984, Nature 312, 604-608; Takeda, et al., 1985, Nature, 314, 452-454) by splicing the genes from a mouse antibody molecule of appropriate antigen specificity together with genes from a human antibody molecule of appropriate biological activity can be used. A chimaeric antibody is a molecule in which different portions are derived from different animal species, such as those having a variable region derived from a murine mAb and a human immunoglobulin constant region. (See, e.g., Cabilly et al., U.S. Pat. No. 4,816,567; and Boss et al., U.S. Pat. No. 4,816397)

In addition, techniques have been developed for the production of humanized antibodies. (See, e.g., Queen, U.S. Pat. No. 5,585,089 .) An immunoglobuin light or heavy chain variable region consists of a "framework" region interrupted by three hypervariable regions, referred to as complementarity determining regions (CDRs). The extent of the framework region and CDRs have been precisely defined (see, "Sequences of Proteins of Immunological Interest", Kabat, E. et al., U.S. Department of Health and Human Services (1983). Briefly, humanized antibodies are antibody molecules from non-human species having one or more CDRs from the non-human species and a framework region from a human immunoglobulin molecule.

Alternatively, techniques described for the production of single chain antibodies (U.S. Pat. No. 4,946,778; Bird, 1988, Science 242, 423-426; Huston, et al., 1988, Proc. Natl. Acad. Sci. USA 85, 5879-5883; and Ward, et al., 1989, Nature 334, 544-546) can be adapted to produce single chain antibodies against NK1 gene products. Single chain antibodies are formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')₂ fragments, which can be produced by pepsin digestion of the antibody molecule and the Fab fragments, which can be generated by reducing the disulfide bridges of the F(ab')₂ fragments. Alternatively, Fab expression libraries may be constructed (Huse, et al., 1989, Science, 246, 1275-1281) to allow rapid and easy identification of monoclonal Fab fragments with the desired specificity.

Also disclosed is a method of determining a predisposition of a subject to ADHD, alcoholism, dyspraxia, conduct disorder, deliberate self harm or injury or suicidality, comprising identifying a polymorphism, mutation or other disruption in the NK1 gene or in the regions flanking the gene in a sample obtained from that subject, wherein the presence of the polymorphism is indicative of a predisposition to ADHD, alcoholism, dyspraxia, conduct disorder, deliberate self harm or injury or suicidality.

The terms polymorphism and mutation are used herein to mean a variation in DNA from the wild-type DNA that results in functioning NK1 receptors. Herein, the term polymorphism preferably refers to a polymorphism that has an effect on the subject's phenotype, specifically a polymorphism that increases susceptibility to ADHD, alcoholism, dyspraxia, conduct disorder, deliberate self harm or injury or suicidality.

The term flanking region refers to the DNA on either side of the NK1 gene. In particular, the term refers to sections that are around 5000 base pairs in length, more preferably around 2500 base pairs, more preferably around 1000 base pairs.

Whilst the polymorphism, mutation or disruption may be in the NK1 gene itself, it may also be in a region that affects the expression of the gene. For example, the polymorphism, mutation or disruption may be in a regulatory region, such as in the NK1 promoter.

The polymorphism may be any type of polymorphism, but is preferably a single nucleotide polymorphism (SNP). It is preferably in the human genome, and is more preferably rs3771856. The polymorphism is preferably at position 75257522 on chromosome 2 and is an A to G switch. This polymorphism is known in the prior art, but its association with ADHD and other conditions has not previously been identified. The inventors have surprising been able to link this polymorphism, that is to say the presence of the G allele with those conditions, especially with alcohol.

A single nucleotide polymorphism is a DNA sequence variation that involves a change in one nucleotide at a particular site.

"Predisposition" is used to mean an increased likelihood of a subject displaying a certain pattern of behaviour compared to a subject without the polymorphism. Preferably it means that the subject is at least 20%, more preferably at least 30%, more preferably at least 40%, more preferably at least 50% more likely to display the pattern of behaviour.

Identification of the SNP may be carried out by methods known in the art, as discussed in the description. In particular polymerase chain reaction (PCR) based methods, comprising amplifying the region of DNA comprising the polymorphism may be used. For example, the polymorphism rs3771856 may be identified using the "Amplifluor" method which uses the polymerase chain reaction to amplify the SNP from the DNA of a case or control combined with a third oligonucleotide labelled fluorimetrically so that it can discriminate between the SNP alleles of rs3771856. Alternatively, it may be rs3771856 genotyped using the "TaqMan assays on demand" method which uses a quencher and a reporter fluorimetric signal on the same oligonucletide to discriminate between alleles.

The subject may be any subject, but is preferably a human. The subject may be displaying characteristics of a particular disease or condition, such as ADHD. Also, the method may be combined with observing the behaviour of the subject, or of the subject's relatives for signs of the condition of interest.

The method may also be used for predicting a subject's likelihood to respond to a particular course of treatment or to predict the likely results of other tests on that subject, such as brain imaging.

A variety of methods can be employed for the diagnostic and prognostic evaluation of NK1 gene disorders and neuropsychiatric disorders, such as conduct disorder, attention deficit hyperactivity disorder and alcoholism, and for the identification of subjects having a predisposition to such disorders.

Such methods may, for example, utilize reagents such as the NK1 gene nucleotide sequences and antibodies directed against NK1 gene products, including peptide fragments thereof. Specifically, such reagents may be used, for example, for:
(1) the detection of the presence of NK1 gene mutations, or the detection of either over- or under-expression of NK1 gene mRNA relative to the state of a NK1 disorder or a neuropsychiatric disorder, such as conduct disorder, attention deficit hyperactivity disorder and alcoholism;
(2) the detection of either an over- or an under-abundance of NK1 gene product relative to the unaffected state; and
(3) the detection of an aberrant level of NK1 gene product activity relative to the unaffected state.

NK1 gene nucleotide sequences can, for example, be used to diagnose an NK1 gene or neuropsychiatric disorder using, for example, the techniques for NK1 gene mutation detection described above.

The methods described herein may be performed, for example, by utilizing prepackaged diagnostic kits comprising at least one specific NK1 gene nucleic acid or anti-NK1 gene antibody reagent described herein, which may be conveniently used, e.g., in clinical settings, to diagnose patients exhibiting abnormalities of a NK1 gene disorder or a neuropsychiatric disorder, such as conduct disorder, attention deficit hyperactivity disorder and alcoholism.

For the detection of NK1 gene mutations, any nucleated cell can be used as a starting source for genomic nucleic acid. For the detection of NK1 gene expression or NK1 gene products, any cell type or tissue in which the NK1 gene is expressed may be utilized.

A variety of methods can be employed to screen for the presence of NK1 gene mutations and to detect and/or assay levels of NK1 gene nucleic acid sequences.

Mutations within the NK1 gene can be detected by utilizing a number of techniques. Nucleic acid from any nucleated cell can be used as the starting point for such assay techniques, and may be isolated according to standard nucleic acid preparation procedures that are well known to those of skill in the art.

NK1 gene nucleic acid sequences may be used in hybridization or amplification assays of biological samples to detect abnormalities involving NK1 gene structure, including point mutations, insertions, deletions, inversions, translocations and chromosomal rearrangements. Such assays may include, but are not limited to, Southern analyses, single-stranded conformational polymorphism analyses (SSCP), and PCR analyses.

Diagnostic methods for the detection of NK1 gene-specific mutations can involve for example, contacting and incubating nucleic acids including recombinant DNA molecules, cloned genes or degenerate variants thereof, obtained from a sample, e.g., derived from a patient sample or other appropriate cellular source, with one or more labelled nucleic acid reagents including recombinant DNA molecules, cloned genes or degenerate variants thereof, under conditions favourable for the specific annealing of these reagents to their complementary sequences within the NK1 gene. Preferably, the lengths of these nucleic acid reagents are at least 15 to 30 nucleotides. After incubation, all non-annealed nucleic acids are removed from the nucleic acid:NK1 gene molecule hybrid. The presence of nucleic acids that have hybridized, if any such molecules exist, is then detected. Using such a detection scheme, the nucleic acid from the cell type or tissue of interest can be immobilized, for example, to a solid support such as a membrane, or a plastic surface such as that on a microtitre plate or polystyrene beads. In this case, after incubation, non-annealed, labelled nucleic acid reagents are easily removed. Detection of the remaining, annealed, labelled NK1 gene nucleic acid reagents is accomplished using standard techniques well-known to those in the art. The NK1 gene sequences to which the nucleic acid reagents have annealed can be compared to the annealing pattern expected from a normal NK1 gene sequence in order to determine whether a NK1 gene mutation is present.

Alternative diagnostic methods for the detection of NK1 gene specific nucleic acid molecules, in patient samples or other appropriate cell sources, may involve their amplification, e.g., by PCR (the experimental embodiment set forth in Mullis, 1987, U.S. Pat. No. 4,683,202), followed by the detection of the amplified molecules using techniques well known to those of skill in the art. The resulting amplified sequences can be compared to those that would be expected if the nucleic acid being amplified contained only normal copies of the NK1 gene in order to determine whether a NK1 gene mutation exists.

Additionally, well-known genotyping techniques can be performed to identify individuals carrying NK1 gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms, which can be utilized for the identification of NK1 gene mutations, have been described that capitalize on the presence of variable numbers of short, tandemly repeated DNA sequences between the restriction enzyme sites. For example, Weber (U.S. Pat. No. 5,075,217) describes a DNA marker based on length polymorphisms in blocks of (dC-dA)n-(dG-dT)n short tandem repeats. The average separation of (dC-dA)n-(dG-dT)n blocks is estimated to be 30,000-60,000 bp. Markers that are so closely spaced exhibit a high frequency co-inheritance, and are extremely useful in the identification of genetic mutations, such as, for example, mutations within the NK1 gene, and the diagnosis of diseases and disorders related to NK1 gene mutations.

Also, Caskey et al. (U.S. Pat. No. 5,364,759) describe a DNA profiling assay for detecting short tri and tetra nucleotide repeat sequences. The process includes extracting the DNA of interest, such as the NK1 gene, amplifying the extracted DNA, and labelling the repeat sequences to form a genotypic map of the individual's DNA.

The level of NK1 gene expression can also be assayed. For example, RNA from a cell type or tissue known, or suspected, to express the NK1 gene, such as brain, may be isolated and tested utilizing hybridization or PCR techniques such as are described, above. The isolated cells can be derived from cell culture or from a patient. The analysis of cells taken from culture may be a necessary step in the assessment of cells to be used as part of a cell-based gene therapy technique or, alternatively, to test the effect of compounds on the expression of the NK1 gene. Such analyses may reveal both quantitative and qualitative aspects of the expression pattern of the NK1 gene, including activation or inactivation of NK1 gene expression.

In one embodiment of such a detection scheme, a cDNA molecule is synthesized from an RNA molecule of interest (e.g., by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (e.g., primers) in the reverse transcription and nucleic acid amplification steps of this method could be prepared by one skilled in the art. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining or by utilizing any other suitable nucleic acid staining method.

Additionally, it is possible to perform such NK1 gene expression assays "in situ", i.e., directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, "PCR In Situ Hybridization: Protocols And Applications", Raven Press, NY).

Alternatively, if a sufficient quantity of the appropriate cells can be obtained, standard Northern analysis can be performed to determine the level of mRNA expression of the NK1 gene.

Antibodies directed against unimpaired or mutant NK1 gene products or conserved variants or peptide fragments thereof, which are discussed, above, may also be used as diagnostics and prognostics for a NK1 gene disorder or a neuropsychiatric disorder, such as conduct disorder, attention deficit hyperactivity disorder and alcoholism, as described herein. Such methods may be used to detect abnormalities in the level of NK1 gene product synthesis or expression, or abnormalities in the structure, temporal expression, and/or physical location of NK1 gene product. The antibodies and immunoassay methods described below have, for example, important in vitro applications in assessing the efficacy of treatments for NK1 gene disorders or neuropsychiatric disorders, such as conduct disorder, attention deficit hyperactivity disorder and alcoholism. Antibodies, or fragments of antibodies, such as those described below, may be used to screen potentially therapeutic compounds in vitro to determine their effects on NK1 gene expression and NK1 gene peptide production. The compounds that have beneficial effects on an NK1 gene disorder or a neuropsychiatric disorder, such as conduct disorder, attention deficit hyperactivity disorder and alcoholism, can be identified, and a therapeutically effective dose determined.

In vitro immunoassays may also be used, for example, to assess the efficacy of cell-based gene therapy for an NK1 gene disorder or a neuropsychiatric disorder, such as conduct disorder, attention deficit hyperactivity disorder and alcoholism. Antibodies directed against NK1 gene peptides may be used in vitro to determine, for example, the level of NK1 gene expression achieved in cells genetically engineered to produce NK1 gene peptides. In the case of intracellular NK1 gene products, such an assessment is done, preferably, using cell lysates or extracts. Such analysis will allow for a determination of the number of transformed cells necessary to achieve therapeutic efficacy in vivo, as well as optimization of the gene replacement protocol.

The tissue or cell type to be analyzed will generally include those that are known, or suspected, to express the NK1 gene. The protein isolation methods employed herein may, for example, be such as those described in Harlow and Lane (1988, "Antibodies: A Laboratory Manual", Cold Spring Harbor Laboratory Press, Cold Spring Harbor, New York). The isolated cells can be derived from cell culture or from a patient. The analysis of cells taken from culture may be a necessary step in the assessment of cells to be used as part of a cell-based gene therapy technique or, alternatively, to test the effect of compounds on the expression of the NK1 gene.

Preferred diagnostic methods for the detection of NK1 gene products or conserved variants or peptide fragments thereof, may involve, for example, immunoassays wherein the NK1 gene products or conserved variants or peptide fragments are detected by their interaction with an anti-NK1 gene product-specific antibody.

For example, antibodies or fragments of antibodies useful in the present invention may be used to quantitatively or qualitatively detect the presence of NK1 gene products or conserved variants or peptide fragments thereof. This can be accomplished, for example, by immunofluorescence techniques employing a fluorescently labeled antibody coupled with light microscopic, flow cytometric, or fluorimetric detection. Such techniques are especially preferred for NK1 gene products that are expressed on the cell surface.

The antibodies (or fragments thereof) useful in the present invention may, additionally, be employed histologically, as in immunofluorescence or immunoelectron microscopy, for in situ detection of NK1 gene products or conserved variants or peptide fragments thereof. In situ detection may be accomplished by removing a histological specimen from a patient, and applying thereto a labelled antibody of the present invention. The antibody (or fragment) is preferably applied by overlaying the labeled antibody (or fragment) onto a biological sample. Through the use of such a procedure, it is possible to determine not only the presence of the NK1 gene product, or conserved variants or peptide fragments, but also its distribution in the examined tissue. Using the present invention, those of ordinary skill will readily perceive that any of a wide variety of histological methods (such as staining procedures) can be modified in order to achieve such in situ detection.

Immunoassays for NK1 gene products or conserved variants or peptide fragments thereof will typically comprise incubating a sample, such as a biological fluid, a tissue extract, freshly harvested cells, or lysates of cells, that have been incubated in cell culture, in the presence of a detectably labeled antibody capable of identifying NK1 gene products or conserved variants or peptide fragments thereof, and detecting the bound antibody by any of a number of techniques well-known in the art.

The biological sample may be brought in contact with and immobilized onto a solid phase support or carrier such as nitrocellulose, or other solid support that is capable of immobilizing cells, cell particles or soluble proteins. The support may then be washed with suitable buffers followed by treatment with the detectably labeled NK1 gene specific antibody. The solid phase support may then be washed with the buffer a second time to remove unbound antibody. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of anti-NK1 gene product antibody may be determined according to well known methods. Those skilled in the art will be able to determine operative and optimal assay conditions for each determination by employing routine experimentation.

One of the ways in which the NK1 gene peptide- specific antibody can be detectably labeled is by linking the same to an enzyme and using an enzyme immunoassay (EIA) (Voller, A., "The Enzyme Linked Immunosorbent Assay (ELISA)", 1978, Diagnostic Horizons 2, 1-7, Microbiological Associates Quarterly Publication, Walkersville, Md.); Voller, A. et al., 1978, J. Clin. Pathol. 31, 507-520; Butler, J. E., 1981, Meth. Enzymol. 73, 482-523; Maggio, E. (ed.), 1980, Enzyme Immunoassay, CRC Press, Boca Raton, Fla.,; Ishikawa, E. et al., (eds.), 1981, Enzyme Immunoassay, Kgaku Shoin, Tokyo). The enzyme which is bound to the antibody will react with an appropriate substrate, preferably a chromogenic substrate, in such a manner as to produce a chemical moiety that can be detected, for example, by spectrophotometric, fluorimetric or by visual means. Enzymes that can be used to detectably label the antibody include, but are not limited to, malate dehydrogenase, staphylococcal nuclease, delta-5-steroid isomerase, yeast alcohol dehydrogenase, α-glycerophosphate, dehydrogenase, triose phosphate isomerase, horseradish peroxidase, alkaline phosphatase, asparaginase, glucose oxidase, β-galactosidase, ribonuclease, urease, catalase, glucose-6-phosphate dehydrogenase, glucoamylase and acetylcholinesterase. The detection can be accomplished by colorimetric methods that employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect NK1 gene peptides through the use of a radioimmunoassay (RIA) (see, for example, Weintraub, B., Principles of Radioimmunoassays, Seventh Training Course on Radioligand Assay Techniques, The Endocrine Society, March, 1986). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase, green fluorescent protein and aequorin.

Further disclosed is the use of a polymorphism in the first intron of the NK1 gene as a marker for a predisposition to ADHD, alcoholism, conduct disorder or suicidality.

Also provided by the invention are a variety of therapeutic approaches. The invention provides the use of a wild-type NK1 gene in the preparation of a medicament for the treatment of ADHD. The invention also provides a wild-type NK1 gene for use in the treatment of ADHD. Similar approaches are disclosed for the related conditions alcoholism, dyspraxia, conduct disorder, deliberate self harm or injury or suicidality.

In a particular embodiment, a vector comprising a wild-type NK1 gene is to be used.

Further provided is the use of a NK1 agonist in the preparation of a treatment for ADHD. Also provided is a NK1 agonist for use in the treatment of ADHD. Similar approaches are disclosed for the related conditions alcoholism, dyspraxia, conduct disorder, deliberate self harm or injury or suicidality.

Further provided is the use of an Angiotensin Converting Enzyme (ACE) inhibitor in the preparation of a treatment for ADHD . Also provided is an ACE inhibitor for use in the treatment of ADHD. Similar approaches are disclosed for the related conditions alcoholism, dyspraxia, conduct disorder, deliberate self harm or injury or suicidality.

An ACE inhibitor is an angiotensin-converting enzyme inhibitor. The term is well known in the art. Without being bound by a particular theory, it is the inventors' understanding that ACE inhibitors enhance NK1 receptor activation by preventing breakdown of substance P in the brain.

Pharmaceutical compositions used in this invention comprise any of the compounds of the present invention, and pharmaceutically acceptable salts and esters thereof, with any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminium stearate, lecithin, serum proteins, such as human serum albumin, buffer substances such as phosphates, glycerine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene-block polymers, polyethylene glycol and wool fat.

The pharmaceutical compositions used in this invention may be administered orally, parenterally, by inhalation spray, rectally, nasally, buccally, vaginally or via an implanted reservoir. Oral administration is preferred. The pharmaceutical compositions of this invention may contain any conventional non-toxic pharmaceutically-acceptable carriers, adjuvants or vehicles. The term parenteral as used herein includes subcutaneous, intracutaneous, intravenous, intramuscular, intra-articular, intrasynovial, intrastemal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

The pharmaceutical compositions of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavouring and/or colouring agents may be added.

The pharmaceutical compositions used in this invention may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions used in this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilising or dispersing agents known in the art.

It may be useful to explore and control the peripheral action of NK1 agonists, by administering an NK1 antagonist that is not able to cross the blood brain barrier. For instance, NK1 agonists *in vitro* increase intestinal motility (de Ahepper et al (2006) Autonom Neurosci 126-127: 273-6;), inhibit oesophogeal contraction (Shiina et al., (2006) Neurosci 139: 495-503) induce stomach relaxation (Muke et L., (2006) Br J Pharmacol 147: 430 -436) increase bronchial secretion (Phillips et al., Br J Pharmacol. (2003) 138: 254-260) and increase myometrial contractions in estrogen-primed mice (Patak et al., Br J Pharmacol (2002) 137:1247-1254). Such actions *in vivo* could present as unwanted side-effects of treatment with an NK1 receptor agonist. They would be prevented by administration of an NK1 antagonist that does not penetrate the brain. Accordingly, when an NK1 agonist is administered for the treatment of ADHD, alcoholism, dyspraxia, conduct disorder, deliberate self harm or injury or suicidality, an NK1 antagonist that does not cross the blood brain barrier may also be administered.

A variety of therapeutic approaches are encompassed by the invention. For example, such methods can comprise administering compounds which modulate the expression of a mammalian NK1 gene and/or the synthesis or activity of a mammalian NK1 gene product so symptoms of the disorder are ameliorated. Alternatively, in those instances in which the neuropsychiatric disorders result from NK1 gene mutations, such methods can comprise supplying the subject with a nucleic acid molecule encoding an unimpaired NK1 gene product such that an unimpaired NK1 gene product is expressed and symptoms of the disorder are reduced or ameliorated.

Symptoms of certain NK1 disorders or neuropsychiatric disorders, such as conduct disorder, attention deficit hyperactivity disorder and alcoholism may be decreased by decreasing the level of abnormal NK1 gene expression and/or abnormal NK1 gene product activity by using NK1 gene sequences in conjunction with well-known antisense, gene "knock-out," ribozyme and/or triple helix methods to decrease the level of abnormal NK1 gene expression. Among the compounds that may exhibit the ability to modulate the activity, expression or synthesis of the NK1 gene, including the ability to ameliorate the symptoms of a NK1 gene disorder or a neuropsychiatric disorder, such as conduct disorder, attention deficit hyperactivity disorder and alcoholism, are antisense, ribozyme, and triple helix molecules. Such molecules may be designed to reduce or inhibit either unimpaired, or if appropriate, mutant target gene activity. Techniques for the production and use of such molecules are well known to those of skill in the art.

Antisense RNA and DNA molecules act to directly block the translation of mRNA by hybridizing to targeted mRNA and preventing protein translation. Antisense approaches involve the design of oligonucleotides that are complementary to a target gene mRNA. The antisense oligonucleotides will bind to the complementary target gene mRNA transcripts and prevent translation. Absolute complementarity, although preferred, is not required.

A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

In one embodiment, oligonucleotides complementary to non-coding regions of the NK1 gene could be used in an antisense approach to inhibit translation of endogenous NK1 gene mRNA. Antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

Regardless of the choice of target sequence, it is preferred that *in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors in vivo), or agents facilitating transport across the cell membrane (see, e.g., Letsinger, et al., 1989, Proc. Natl. Acad. Sci. U.S.A. 86,6553-6556; Lemaitre, et al., 1987, Proc. Natl. Acad. Sci. 84, 648-652; PCT Publication No. WO88/09810, published Dec. 15, 1988) or the blood-brain barrier (see, e.g., PCT Publication No. WO89/10134, published Apr. 25, 1988), hybridization-triggered cleavage agents (see, e.g., Krol et al., 1988, BioTechniques 6, 958-976) or intercalating agents (see, e.g., Zon, 1988, Pharm. Res. 5, 539-549). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

The antisense oligonucleotide may comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

The antisense oligonucleotide may be an alpha-anomeric oligonucleotide. An alpha-anomeric oligonucleotide forms specific double-stranded hybrids with complementary RNA in which, contrary to the usual β-units, the strands run parallel to each other (Gautier, et al., 1987, Nucl. Acids Res. 15, 6625-6641). The oligonucleotide is a 2'-O-methylribonucleotide (Inoue, et al., 1987, Nucl. Acids Res. 15, 6131-6148), or a chimeric RNA-DNA analogue (Inoue, et al., 1987, FEBS Lett. 215, 327-330).

Oligonucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein, *et al.* (1988, *Nucl. Acids Res.* 16, 3209), methylphosphonate oligonucleotides can be prepared by use of controlled pore glass polymer supports (Sarin, et al., 1988, Proc. Natl. Acad. Sci. U.S.A. 85, 7448-7451), etc.

While antisense nucleotides complementary to the target gene coding region sequence could be used, those complementary to the transcribed, untranslated region are most preferred.

Antisense molecules should be delivered to cells that express the target gene in vivo. A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

The ribozymes of the present invention also include RNA endoribonucleases (hereinafter "Cech-type ribozymes") such as the one that occurs naturally in *Tetrahymena thermophila* (known as the IVS, or L-19 IVS RNA) and that has been extensively described by Thomas Cech and collaborators (Zaug, et al., 1984, Science, 224, 574-578; Zaug and Cech, 1986, Science, 231, 470-475; Zaug, et al., 1986, Nature, 324, 429-433; published International patent application No. WO 88/04300 by University Patents Inc.; Been and Cech, 1986, Cell, 47, 207-216). The Cech-type ribozymes have an eight base pair active site which hybridizes to a target RNA sequence whereafter cleavage of the target RNA takes place.

As in the antisense approach, the ribozymes can be composed of modified oligonucleotides (e.g., for improved stability, targeting, etc.) and should be delivered to cells that express the target gene in vivo. A preferred method of delivery involves using a DNA construct "encoding" the ribozyme under the control of a strong constitutive pol III or pol II promoter, so that transfected cells will produce sufficient quantities of the ribozyme to destroy endogenous target gene messages and inhibit translation. Ribozymes, unlike antisense molecules, are catalytic, and hence a lower intracellular concentration is required for efficiency.

Alternatively, endogenous target gene expression can be reduced by targeting deoxyribonucleotide sequences complementary to the regulatory region of the target gene (i.e., the target gene promoter and/or enhancers) to form triple helical structures that prevent transcription of the target gene in target cells in the body. (See generally, Helene, 1991, Anticancer Drug Des., 6(6), 569-584; Helene, et al., 1992, Ann. N.Y. Acad. Sci., 660, 27-36; and Maher, 1992, Bioassays 14(12), 807-815).

Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC⁺ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

In instances wherein the antisense, ribozyme, and/or triple helix molecules described herein are utilized to inhibit mutant gene expression, it is possible that the technique may so efficiently reduce or inhibit the transcription (triple helix) and/or translation (antisense, ribozyme) of mRNA produced by normal target gene alleles that the possibility may arise wherein the concentration of normal target gene product present may be lower than is necessary for a normal phenotype. In such cases, to ensure that substantially normal levels of target gene activity are maintained, therefore, nucleic acid molecules that encode and express target gene polypeptides exhibiting normal target gene activity may, be introduced into cells via gene therapy methods such as those described that do not contain sequences susceptible to whatever antisense, ribozyme, or triple helix treatments are being utilized. Alternatively, in instances whereby the target gene encodes an extracellular protein, it may be preferable to co-administer normal target gene protein in order to maintain the requisite level of target gene activity.

Anti-sense RNA and DNA, ribozyme, and triple helix molecules of the invention may be prepared by any method known in the art for the synthesis of DNA and RNA molecules, as discussed above. These include techniques for chemically synthesizing oligodeoxyribonucleotides and oligoribonucleotides well known in the art such as for example solid phase phosphoramidite chemical synthesis. Alternatively, RNA molecules may be generated by *in vitro and in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

With respect to an increase in the level of normal NK1 gene expression and/or NK1 gene product activity, NK1 gene nucleic acid sequences can, for example, be utilized for the treatment of a NK1 gene disorder or a neuropsychiatric disorder, such as conduct disorder, attention deficit hyperactivity disorder, self harm and injury, suicidality and alcoholism. Such treatment can be administered, for example, in the form of gene replacement therapy. Specifically, one or more copies of a normal NK1 gene or a portion of the NK1 gene that directs the production of a NK1 gene product exhibiting normal NK1 gene function, may be inserted into the appropriate cells within a patient, using vectors that include, but are not limited to adenovirus, adeno-associated virus, and retrovirus vectors, in addition to other particles that introduce DNA into cells, such as liposomes.

The NK1 gene is expressed in the brain and so such gene replacement therapy techniques should be capable of delivering NK1 gene sequences to these cell types within patients. Thus, in one embodiment, techniques that are well known to those of skill in the art (see, e.g., PCT Publication No. WO89/10134, published Apr. 25, 1988) can be used to enable NK1 gene sequences to cross the blood-brain barrier readily and to deliver the sequences to cells in the brain. With respect to delivery that is capable of crossing the blood-brain barrier, viral vectors such as, for example, those described above, are preferable. Also included are methods using liposomes either *in vivo*, *ex vivo* or *in vitro,* wherein NK1 gene sense or antisense DNA is delivered to the cytoplasm and nucleus of target cells. Liposomes can deliver NK1 gene sense or antisense RNA to humans and the human brain or in mammals through intrathecal delivery either as part of a viral vector or as DNA conjugated with nuclear localizing proteins or other proteins that increase take up into the cell nucleus.

In another embodiment, techniques for delivery involve direct administration of such NK1 gene sequences to the site of the cells in which the NK1 gene sequences are to be expressed. Additional methods that may be utilized to increase the overall level of NK1 gene expression and/or NK1 gene product activity include the introduction of appropriate NK1 GENE-expressing cells, preferably autologous cells, into a patient at positions and in numbers that are sufficient to ameliorate the symptoms of a NK1 gene disorder or a neuropsychiatric disorder, such as conduct disorder, attention deficit hyperactivity disorder and alcoholism. Such cells may be either recombinant or non-recombinant.

Among the cells that can be administered to increase the overall level of NK1 gene expression in a patient are normal cells, preferably brain cells and also choroid plexus cells within the CNS which are accessible through intrathecal injections. Alternatively, cells, preferably autologous cells, can be engineered to express NK1 gene sequences, and may then be introduced into a patient in positions appropriate for the amelioration of the symptoms of a NK1 gene disorder or a neuropsychiatric disorder, such as conduct disorder, attention deficit hyperactivity disorder and alcoholism. Alternately, cells that express an unimpaired NK1 gene and that are from a MHC matched individual can be utilized, and may include, for example, brain cells. The expression of the NK1 gene sequences is controlled by the appropriate gene regulatory sequences to allow such expression in the necessary cell types. Such gene regulatory sequences are well known to the skilled artisan. Such cell-based gene therapy techniques are well known to those skilled in the art, see, e.g., Anderson, U.S. Pat. No. 5,399,349.

When the cells to be administered are non-autologous cells, they can be administered using well known techniques that prevent a host immune response against the introduced cells from developing. For example, the cells may be introduced in an encapsulated form which, while allowing for an exchange of components with the immediate extracellular environment, does not allow the introduced cells to be recognized by the host immune system.

Additionally, compounds, such as those identified via techniques such as those described, that are capable of modulating NK1 gene product activity can be administered using standard techniques that are well known to those of skill in the art. In instances in which the compounds to be administered are to involve an interaction with brain cells, the administration techniques should include well known ones that allow for a crossing of the blood-brain barrier such as intrathecal injection and conjugation with compounds that allow transfer across the blood brain barrier.

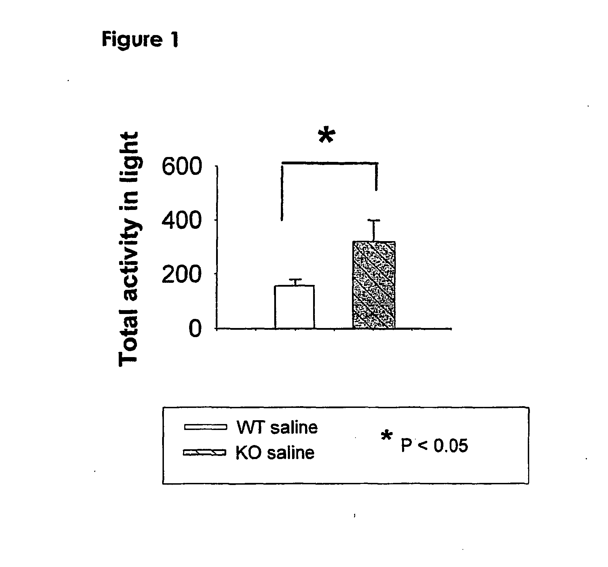

The invention will now be described in detail by way of example only, with references to the figures, in which
Figure 1 shows hyperactivity in NK1-/- mice in a light / dark exploration box. Effect of NK1 genotype on locomotor activity scored as the number of lines crossed in a light / dark exploration box. Values show mean ± s.e. mean. N = 7 per group. * P < 0.05.
Figure 2 shows hyperactivity of NK1-/- mice in an activity chamber and its reduction by DMI (a drug used to treat ADHD). Values show mean ± s.e. mean. N = 6 per group. Vehicle = water. ** <P < 0.01 for comparisons of groups indicated by the bars (one-way ANOVA and *post hoc* test). Data also confirm that desipramine (DMI), which is a drug treatment for ADHD, reduces locomotor activity in NK1R-/- mice but not NK1R+/+ mice.
Figure 3 shows hyperactivity of NK1-/- mice in a light / dark exploration box and its reduction by DMI.

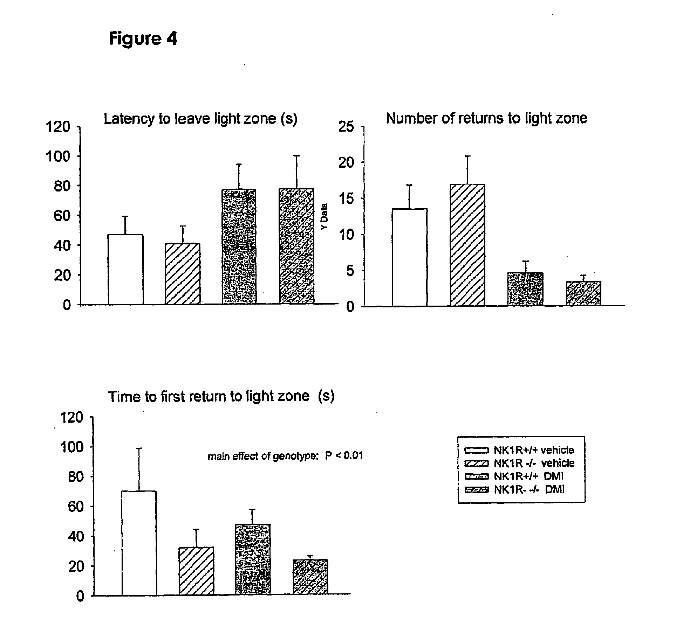

Figure 4 shows impulsivity in vehicle-injected NK1-/- mice.
Figure 5 shows a comparison of the activity of wildtype and knockout mice when given d-AMP or methylphenidate.

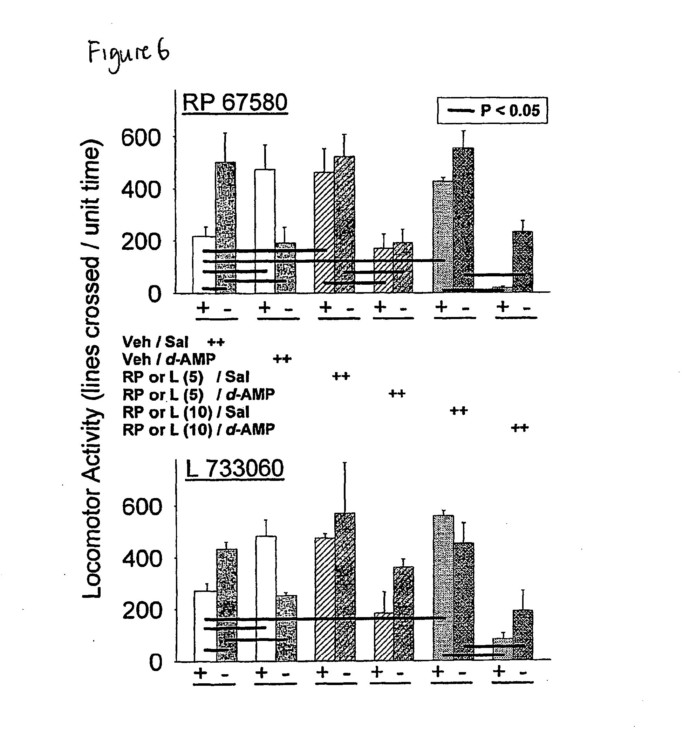

Figure 6 shows the effect of NK1R antagonists RP67580 or L 733060 and d-AMP on locomotor activity. Locomotor activity of NK1R+/+ mice (+) and NKtR-/- mice (-) following administration of saline (Sal) or d-amphetamine (dAMP: 2.5 mg/kg i.p.). These two treatments were assigned to mice that had been pretreated with either vehicle (Veh: Tween 80 in 0.9% saline) or an NK1R antagonist (RP 67580 ('RP') or L 733060 ('L')) at the doses indicated in parentheses (5 or 10 mg/kg i.p). Bars show mean ± s.e. mean locomotor activity in the light zone of the LDEB per unit time. Lines linking pairs of treatment groups indicate differences between the means at P ≤ 0.05 (or less).
Figure 7 shows that NK1-/- mice have impaired motor coordination.
Figure 8 illustrates the reduced dopamine efflux seen in the frontal cortex of NK1-/- mice.
Figure 9 illustrates the increased noradrenaline efflux seen in the frontal cortex of NK1-/- mice.
Figure 10 illustrates that there is no d-AMP induced increase in dopamine efflux in NK1-/- mice.
Figure 11 illustrates the desensitization of α2_{A}-adrenoreceptors on noradrenergic neurons in NK1-/- mice.
Figure 12 illustrates the disruption in predicted dependence on d-AMP and morphine seen in NK1-/- mice.
Figure 13 illustrates the effect of beta-adrenoreceptor blockers on hyperactivity in NK1^{-/-} mice.

As shown in Table 1, the inventors have identified the core features in ADHD and found corresponding features in NK1^{-/-} mice. Certain features have been identified previously, as mentioned in the table, but the majority of the features have not been identified previously, nor have they been linked with ADHD.

### EXAMPLES

### LOCOMOTOR ACTIVITY AND IMPULSIVITY

### Light Dark Exploration Box Protocol

### 1. Subjects

Experiments were carried out on male NK1-/- ('knock-out') and NK1+/+ (wild-type) mice, weighing 25-31 g (*i.e*. about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

They were housed in groups of two to five per cage under a 12:12 h light/dark cycle (lights on at 08:00 h). Temperature and relative humidity were controlled at 21 ±2°C and 45 ± 5%, respectively. Food and water were freely available. The genotype of each animal was verified *post mortem,* using DNA isolated from the animal's tail tip, and PCR.

### 1. Behavioural procedure

All experiments were performed in treatment-naïve mice, between 13:00 h and 17:00 h. Behavioural tests were carried out in a custom-built light/dark exploration box (LDEB) (length 45 cm; width 20 cm; depth 25 cm). The test box consisted of a small 'dark zone' (length 15 cm; 4 lux) and a larger 'light zone' (length 30 cm; 20 lux). The walls and floor of the 'dark zone' were coloured black. The walls and floor of the 'light zone' were coloured white. The floor of both zones was marked as a grid of 5 x 5 cm squares (12 in the dark zone, 24 in the light zone), which was used to score locomotor activity. The partition separating the two zones incorporated a small guillotine-style door (height 10.5 cm, width 6.5 cm), which could be raised to allow animals to commute between the two compartments.

Both genotypes of mice were randomly assigned to one of two treatment groups (desipramine or vehicle; N=7 per group). Individual mice were first habituated, for 90 min, to the dark zone of the exploration box, with the guillotine door closed. After the first 60 min, each animal received an i.p. injection (10 mL/kg) of [either desipramine (10 mg/kg)] or vehicle (H₂O) and then replaced in the dark zone for a further 30 min. The mouse was then transferred, with minimal handling, to the centre of the novel, white zone (facing the wall). At the same time, the guillotine door was raised to enable the mouse to commute between the two zones. The behaviour of the mouse was recorded on video for the following 30 min. The following behaviours were later scored 'blind', in 10 min time-bins:
i) latency to leave the light zone following forced entry (anxiety-like behaviour or impulsivity?)
ii) latency to first return to the light zone following the first exit (the appearance of all four paws was the criterion for re-entry) (impulsivity)
iii) number of returns to the light zone (impulsivity)
iv) number of lines crossed by all four paws of the mouse (as an index of locomotor activity)

### 3. Data analysis

Data were analysed by two-way analysis of variance (ANOVA) using 'genotype' and 'treatment' as main factors. Then, one-way ANOVA, with 'group' as the factor was carried out followed by the Tukey *post hoc* test to compare pairs of data. Analysis of covariance (ANCOVA) was used to control for any effects of locomotor activity on other behavioural measures, using locomotor activity as a covariate in the analysis. Data are expressed as means ± s.e.mean and P≤0.05 was set as the criterion for significance.

The NK1-/- mice showed increased locomotor activity, especially in the light area, suggesting hyperactivity and reduced latency to return to the light area, suggesting impulsivity when compared to the NK1+/+ mice. This is shown in Figures, 1, 3 and 4.

### Activity Meter Protocol

Behavioural tests were carried out in a standard locomotor activity chamber comprising a custom-made transparent perspex box (length 30 cm; width 19 cm; depth 18 cm). The box was placed in the centre of an activity meter (410 x 410 mm, Columbia Instruments, USA) which was equipped with parallel infrared beams spanning the chamber. Ambulatory activity was scored whenever the mouse interrupted two adjacent beams. Light intensity within the chamber was 82 lux.

All experiments were performed in drug-naive mice, between 14:00 h and 18:30 h. Both genotypes received an i.p. injection (10 mL/kg) of vehicle (saline) and then replaced in its home cage. 30 min later, the mouse was transferred, with minimal handling, to the centre of the locomotor activity chamber and scored for ambulatory activity. The apparatus was cleaned thoroughly with water after each experiment.

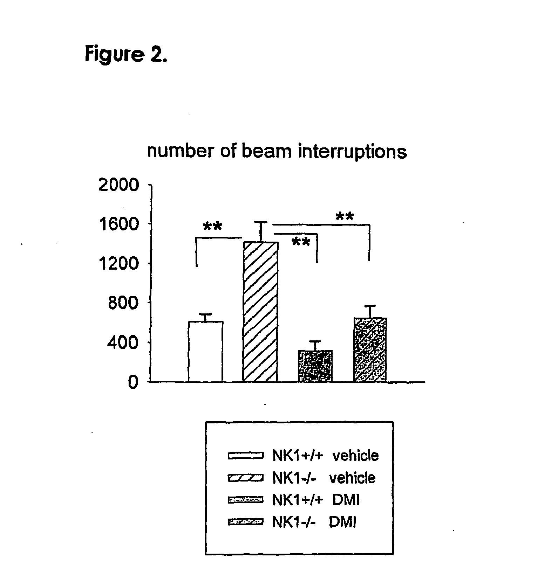

Figure 2 shows the number of beam interruptions for each phenotype. As can be seen, the NK1-/- mice interrupted the beam more than the wild-type mice, suggesting hyperactivity.

### LOCOMOTOR ACTIVITY IS REDUCED BY d-AMP IN NK1-/- MICE BUT INCREASED IN NK1+/+ MICE and THE BEHAVIOUR OF NK1+/+ MICE GIVEN A NK1 ANTAGONIST (RP 67580 OR L733060) ('PSEUDO KNOCKOUT') MIMICS THAT OF NK1-/- MICE

### Protocol

### 1. Subjects

Experiments were carried out on male NK1-/- ('knock-out') and NK1+/+ (wild-type) mice, weighing 25-35 g (*i.e.* about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

The mice were housed in groups of two to five per cage under a 12:12 h light/dark cycle (lights on at 08:00 h). Temperature and relative humidity were controlled at 21 ± 2°C and 45 ± 5%, respectively. Food and water were freely available. At the end of every experiment, a tissue sample was taken from the tail-tip of each animal for verification of genotype *post mortem* by PCR.

### Behavioural procedures

All experiments tested the behaviour of (formerly) treatment-naïve mice in the LDEB and were carried out between 13:00 h and 15:00 h. Each LDEB consisted of a small 'dark zone' (length, 15 cm; width, 20 cm; 174 lux) and a larger 'light zone' (length 30 cm; width, 20 cm; 346 lux). The walls and floor of the 'dark zone' were coloured black The walls and floor of the 'light zone' were coloured white. The floor of both zones was marked as a grid of 5 x 5 cm squares (12 in the dark zone, 24 in the light zone) which was used to score locomotor activity. The partition separating the two zones incorporated a small (white) guillotine-style door (height 10.5 cm, width 7.5 cm), which could be raised to allow animals to commute between the two compartments. Mice were tested in pairs in two LDEBs, placed side by side. At 13:00h, each mouse was placed in the dark zone of a LDEB, with the partition door closed. After 30 min in the dark zone, mice of each genotype were randomly assigned to one of six treatment groups (n=3~4) and given an injection (10 mL/kg; i.p) of either vehicle (one drop of Tween 80 in saline to appropriate volume) or the NK1 antagonist, RP 67580 (5 or 10 mg/kg). The same subjects were given a second injection or either saline or *d*-amphetamine (2.5 mg/kg, i.p), 30 min later.

For each genotype, the treatment groups were:

| **Injection 1** | **/ Injection 2** |
|---|---|
| -- Vehicle | / Vehicle |
| -- RP 67580 (5) | / Vehicle |
| -- RP 67580 (10) | / Vehicle |
| -- Vehicle | / *d*-AMP |
| -- RP 67580 (5) | / *d*-AMP |
| -- RP 67580 (10) | / *d*-AMP |

At 14:30h, each mouse was transferred to the centre of the novel light zone, facing away from the guillotine style door. The door was immediately lifted to allow the animals to commute freely between the two compartments. Their behaviour was recorded with a Sony Handycam Vision video recorder for 30 min. The number of lines crossed by all four paws of the mouse (as an index of locomotor activity) was scored 'blind', in 5-min time-bins.

In a second experiment, this protocol was repeated, substituting the NK1 antagonist, L 733060 (5 mg/kg) in place of RP 67580. All other details (including Tween vehicle) were the same.

### 3. Drugs and reagents

RP 67580 was administered at 5 or 10 mg/kg; dissolved in a drop of Tween 80 and adjusted to the appropriate concentration by adding 0.9% saline. The same mixture was used as vehicle for the first injection of control mice. *d*-AMP (2.5 mg/kg) (Sigma, Poole, UK) was dissolved in 0.9% saline which was used as the vehicle for the second injection. RP 67580 and L733060 were purchased from Tocris (Avonmouth, UK).

### 4. Data analysis

The data were analysed by three-way (experiment testing RP 67580) or two-way (for L 733050) ANOVA using 'genotype', 'drug' and 'dose' (if appropriate) as main factors. Then, one-way ANOVA, with 'group' as the factor was carried out followed by the Tukey *post hoc* test to compare group pairs. Data are expressed as means ± s.e.mean and *P*≤0.05 was set as the criterion for significance.

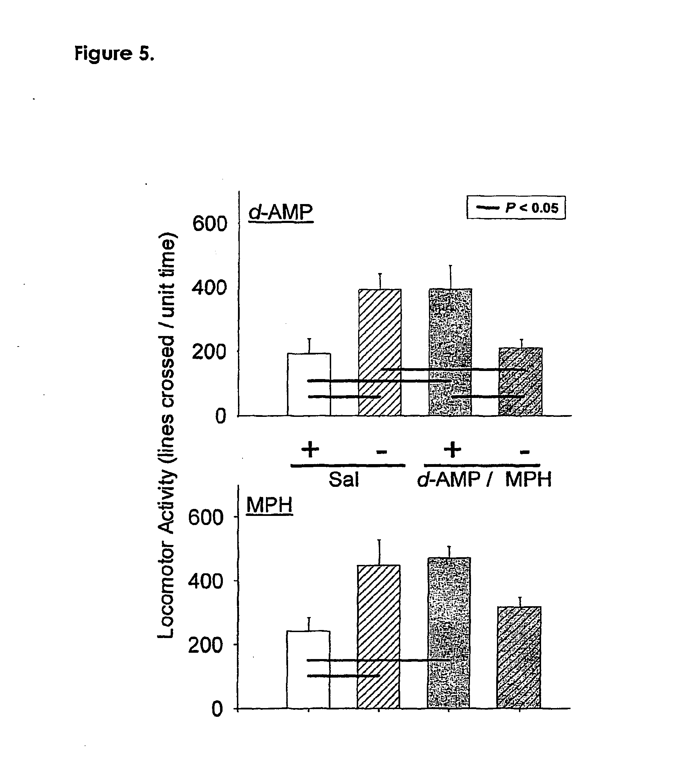

As may be seen from figures 5 and 6 locomotor activity is reduced by d-AMP in NK1-/- mice, but increased in NK1+/+ mice, mimicking the effect d-AMP has in humans with and without ADHD.

### LOCOMOTOR ACTIVITY IS REDUCED BY METHYLPHENIDATE IN NK1-/- MICE BUT INCREASED IN NK1+/+ MICE.

### Protocol

### 1. Subjects

Experiments were carried out on male NK1-/- ('knock-out') and NK1+/+ (wild-type) mice, weighing 25-35 g (*i.e.* about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

The mice were housed in groups of two to five per cage under a 12:12 h light/dark cycle (lights on at 08:00 h). Temperature and relative humidity were controlled at 21 ± 2°C and 45 ± 5%, respectively. Food and water were freely available. At the end of every experiment, a tissue sample was taken from the tail-tip of each animal for verification of genotype *post mortem* by PCR.

### 2. Behavioural procedures

All experiments tested the behaviour of (formerly) treatment-naïve mice in the LDEB and were carried out between 13:00 h and 15:00 h. Each LDEB consisted of a small 'dark zone' (length, 15 cm; width, 20 cm; 174 lux) and a larger 'light zone' (length, 30 cm; width 20 cm; 346 lux). The walls and floor of the 'dark zone' were coloured black. The walls and floor of the 'light zone' were coloured white. The floor of both zones was marked as a grid of 5 x 5 cm squares (12 in the dark zone, 24 in the light zone) which was used to score locomotor activity. The partition separating the two zones incorporated a small (white) guillotine-style door (height 10.5 cm, width 7.5 cm), which could be raised to allow animals to commute between the two compartments. Mice were tested in pairs in two LDEBs, placed side by side. At 13:00h, each mouse was placed in the dark zone of a LDEB, with the partition door closed. After 60 min in the dark zone, the mice were randomly assigned to one of two treatment groups for each genotype (n=9 per group) and were given an injection (10 mL/kg; i.p) of either vehicle (saline) or methylphenidate (2.5 mg/kg, i.p). 30 min later, the mice were transferred to the centre of the novel light zone, facing away from the guillotine style door. The door was immediately lifted to allow the animals to commute freely between the two compartments. Their behaviour was recorded with a Sony Handycam Vision video recorder for 30 min. The number of lines crossed by all four paws of the mouse (an index of locomotor activity) was scored 'blind', in 10-min time-bins.

### 3. Drugs and reagents

Vehicle was 0.9% saline (Sigma, Poole, U.K). Methylphenidate was obtained from (Sigma, Poole, UK) and administered at 2.5 mg/kg (i.p. 10 ml.kg); dissolved in 0.9% saline.

### 4. Data analysis

The data were analysed by two-way ANOVA using `genotype' and 'treatment as main factors. Then, one-way ANOVA, with 'group' as the factor was carried out followed by the Tukey *post hoc* test to compare pairs of data. Data are expressed as means ± s.e.mean and *P*≤0.05 was set as the criterion for significance.

Figures 5 illustrates the increased locomotor activity seen in NK1+/++ mice and reduced activity seen in NK1-/- mice following administration of methylphenidate. as in previous experiments, the reduced latency to leave or return ot the light zone is suggestive of impulsivity.

### IMPAIRED MOTOR CO-ORDINATION AND LEARNING ON THE ROTAROD

### 1. Protocol

Mice were placed individually on the rotating drum of a Rota-Rod Treadmill (for mice: Ugo-Basile, Comerio, Italy) set to turn at a fixed speed (10, 20, 40 rpm), or to accelerate from 5 to 40 rpm, for 3 min. The time taken by each mouse to fall was recorded. Each mouse underwent 10 trials on the rota-rod per day.

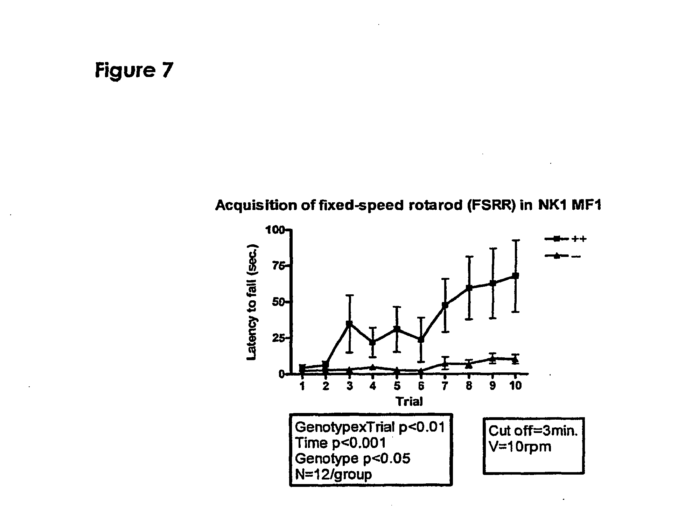

As may be seen from figure 7 the NK1-/- mice showed impaired co-ordination and learning.

### REDUCED BASAL EXTRACELLULAR DOPAMINE IN THE FRONTAL CORTEX OF FREELY-MOVING NK1-/- MICE

### Protocols

### 1. Subjects

Experiments were carried out on male NK1-/- ('knock-out') and NK1+/+ (wild-type) mice, weighing 25-35 g (*i.e.* about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

The mice were housed in groups of two to five per cage under a 12:12 h light/dark cycle (lights on at 08:00 h). Temperature and relative humidity were controlled at 21 ± 2°C and 45 ± 5%, respectively. Food and water were freely available. A tissue sample from the tail tip of each animal was taken for verification of genotype, *postmortem*, by PCR.

### 2 Surgical procedures and microdialysis

Microdialysis probes were constructed in-house and equipped with cuprophan membrane (inner diameter 200 µm, outer diameter 250 µm, molecular weight cut-off 5 kD; Medicell International Ltd., England) forming an active dialysis window of 2 mm. Anaesthesia was induced in a closed chamber delivering 2.0% halothane combined with 95% O₂ / 5% CO₂ at 2 L/min. The mice were then placed in a stereotaxic frame and anaesthesia maintained, via a face mask, with 1.5% halothane in 95% O₂ / 5% CO₂ (2 L/min).

A small incision was made in the scalp to expose the skull and reveal bregma. Then, following craniotomy, a microdialysis probe, primed with modified Ringer's solution (NaCI 145 mM, KCI 4 mM, CaCl₂ 1.3 mM, pH 6.8), was implanted into the frontal cortex (AP +2.1 mm, ML +1.0 mm, DV -2.0 mm). Vital signs were monitored continually to ensure a respiratory rate of 100-190/min (average: 135 ± 20/min). Core body temperature was maintained at 38°C using a warm water-bed.

After implantation, the probe was anchored to the skull with dental cement and the mice then allowed to recover from the anaesthesia, overnight. Microdialysis was carried out the next day during which probes were perfused with modified Ringer's solution at a rate of 1.5 µL/min. After a washout (equilibration) period of 1.5 h, dialysis samples collected at 20-min intervals and a stable efflux for at least three consecutive samples was taken as the baseline.

### 3. HPLC and electrochemical detection

The dopamine in the dialysis samples was separated by reversed-phase ion-pair chromatography on a Hypersil ODS 5 µm column (250 x 4.6 mm) maintained at room temperature and protected by an Aquapore guard column (30 x 4.6 mm). The mobile phase contained 0.23mM octane sulphonic acid, 83 mM sodium dihydrogen orthophosphate, 0.84 mM EDTA, 17% methanol, adjusted to pH 4.0 and was pumped through the system at 1.0 mL/min. Dopamine in the sample was detected using a Coulochem II detector (ESA) and the mobile phase was conditioned by a guard cell (ESA model 5020). Chromatograms were processed using a Spectraphysics Chromjet integrator. The dopamine content of the samples was calculated from the peak height of the chromatogram with reference to external standards. There was no correction for probe recovery.

### 4. Drugs and reagents

Halothane was purchased from University College Hospital (Pharmacy). Buffer reagents were either AnalaR or HPLC grade and purchased from BDH / VWR.

### 5. Data analysis

Raw data from the microdialysis were analysed using repeated measures ANOVA (SPSS PC⁺). The significance of the difference in dopamine efflux was assessed using repeated measures two-way ANOVA in which 'time' was treated as a 'within subjects' factor and 'genotype' was treated as a 'between subjects' factor. The Greenhouse - Geisser 'ε' correction was applied to correct for any violation of sphericity of the variance - covariance matrix. The criterion for statistical significance was set at *P*≤0.05.

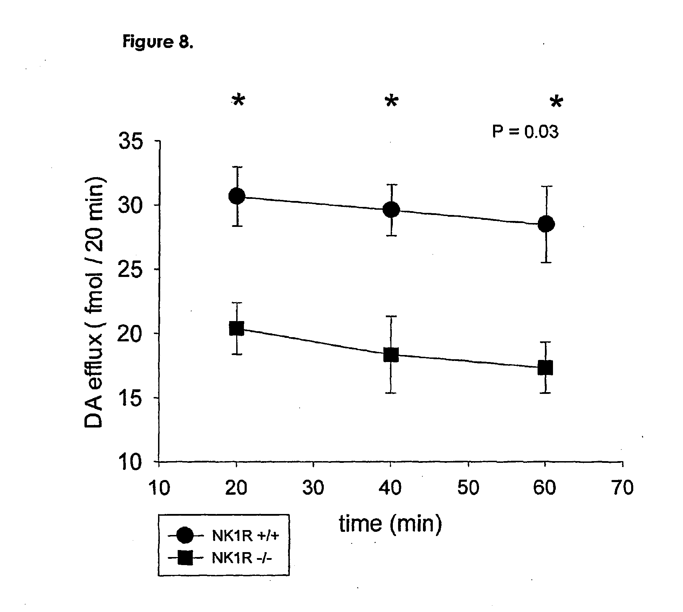

As seen in figure 8, NK1-/- mice showed reduced basal efflux of dopamine when compared to NK1+/+ mice.

### INCREASED BASAL AFFLUX OF NORADRENALINE IN THE FRONTAL CORTEX OF NK1-/-MICE

### Protocol

### 1. Subjects

Experiments were carried out on male NK1-/- ('knock-out') and NK1+/+ (wild-type) mice, weighing 25-31 g (*i.e.* about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

The mice were housed in groups of two to five per cage under a 12:12 h light/dark cycle (lights on at 08:00 h). Temperature and relative humidity were controlled at 21 ± 2°C and 45 ± 5%, respectively. Food and water were freely available. At the end of every experiment, a tissue sample was taken from the tail-tip of each animal for verification of genotype *post mortem* by PCR.

### 2 Surgical procedures and microdialysis

Microdialysis probes were constructed in-house and were equipped with cuprophan membrane (inner diameter 200 µm, outer diameter 250 µm, molecular weight cut-off 5 kD; Medicell International Ltd., England) forming an active dialysis window of 1.5 mm. Anaesthesia was induced in a closed chamber delivering 2.0% halothane combined with 30% O₂ / 70% N₂ at 2 L/min. The mice were then placed in a stereotaxic frame and anaesthesia maintained, via a face mask, with 1.5% halothane in 30% O₂ / 70% N₂ (2 L/min).

A small incision was made in the scalp to expose the skull and reveal bregma. Then, following craniotomy, a microdialysis probe, primed with modified Ringer's solution (NaCI 145 mM, KCI 4 mM, CaCl₂ 1.3 mM, pH 6.8), was implanted into the M2 region of the cerebral cortex (AP +2.1 mm, ML ±1.0 mm, DV -2.0 mm).

After probe implantation, the mice were maintained under anaesthesia for the duration of the experiment. Vital signs were monitored continually to ensure a respiratory rate of 100-190/min (average: 135 ± 20/min). Core body temperature was maintained at 38°C using a warm water-bed. Sterile saline (0.9%, 0.1 mL) was injected subcutaneously, once per hour, to prevent dehydration.

### 3. Microdialysis

Microdialysis probes were perfused with modified Ringer's solution at a rate of 1.5 µL/min and dialysis samples collected at 20-min intervals, starting 20 - 40 min after implantation. Stable efflux over a minimum of three consecutive samples was taken as baseline. At the end of each experiment, mice were killed by an overdose of anaesthetic, and cervical dislocation, followed immediately by removal of the brain, which was stored in 10% formalin at 4°C. The correct position of the probe was confirmed in coronal sections by reference to the atlas by Paxinos and Franklin (2001). It was not necessary to exclude any data on the basis of incorrect probe implantation.

### 4. HPLC and electrochemical detection

The noradrenaline content of the dialysis samples was determined by HPLC coupled to electrochemical detection. Solutes were separated by reversed-phase ion-pair chromatography on a Hypersil ODS 5 µm column (250 x 4.6 mm) maintained at room temperature and protected by an Aquapore guard column (30 x 4.6 mm). The mobile phase contained 2mM octane sulphonic acid, 100 mM sodium dihydrogen orthophosphate, 0.67 mM EDTA, 12% methanol, adjusted to pH 3.75 and was pumped through the system at 1.0 mL/min. Noradrenaline was detected using a Coulochem II detector (ESA) and the mobile phase was conditioned by a guard cell (ESA model 5020). Chromatograms were processed using Turbochrome 4.1 software (Perkin Elmer, USA). The noradrenaline content of the samples was calculated from the peak height of the chromatogram with reference to an external standard. There was no correction for probe recovery.

### 5. Drugs and reagents

Halothane was purchased from Rhodia (Bristol, U.K.). Buffer reagents were either AnalaR or HPLC grade.

### 6. Data analysis

Raw data from the microdialysis were analysed using repeated measures ANOVA (SPSS PC⁺). The significance of the difference in noradrenaline efflux was assessed using repeated measures two-way ANOVA in which 'time' was treated as a 'within subjects' factor and 'genotype' was treated as a 'between subjects' factor. The Greenhouse - Geisser 'ε' correction was applied to correct for any violation of sphericity of the variance - covariance matrix. The criterion for statistical significance was set at *P*≤0.05.

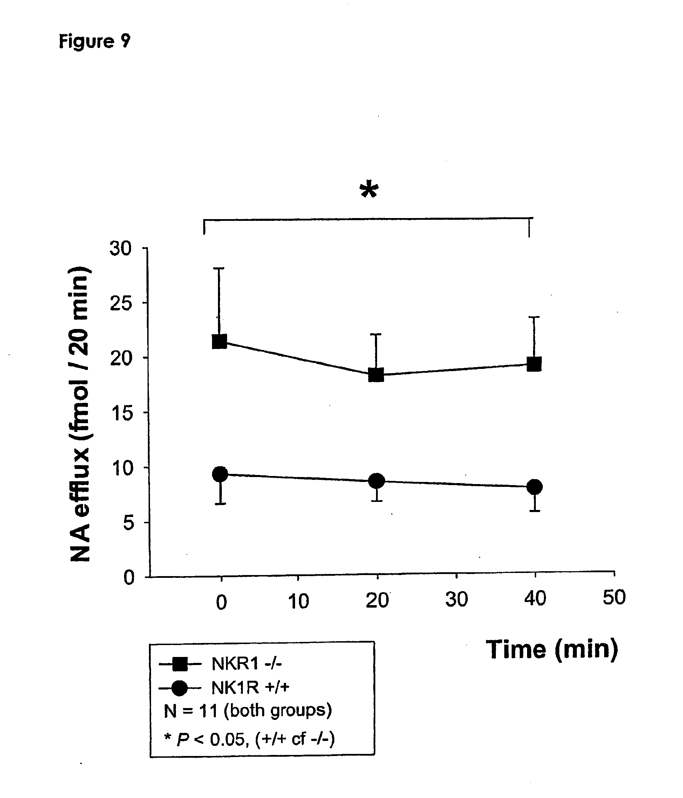

Figure 9 illustrates that NK1-/- mice were found to have increased efflux of noradrenaline in the frontal cortex.

### d-AMPHETAMINE INCREASES EXTRACELLULAR DOPAMINE IN THE FRONTAL CORTEX (M1) / DORSAL STRIATUM OF NK1+/+, BUT NOT NK1-/-, MICE

### Protocol

### 1. Subjects

Experiments were carried out on male NK1-/- ('knock-out') and NK1+/+ (wild-type) mice, weighing 25-35 g (*i.e.* about 6-7 weeks of age). Mice were derived from a 129/Sv X C57BL/6 genetic background that had been crossed with an outbred MF1 strain (Harlan OLAC, Bicester, UK). All mice were taken from a colony maintained at University College London.

The mice were housed in groups of two to five per cage under a 12:12 h light/dark cycle (lights on at 08:00 h). Temperature and relative humidity were controlled at 21 ± 2°C and 45 ± 5%, respectively. Food and water were freely available. A tissue sample was taken from each animal's tail tip for *post mortem* verification of genotype by PCR.

### 2. Surgical procedures and microdialysis

Microdialysis probes were constructed in-house and equipped with cuprophan membrane (inner diameter 200 µm, outer diameter 250 µm, molecular weight cut-off 5 kD; Medicell International Ltd., England) forming an active dialysis window of 3.5 mm. Anaesthesia was induced in a closed chamber delivering 2.0% halothane combined with 95% O₂ / 5% CO₂ at 2 L/min. The mice were then placed in a stereotaxic frame and anaesthesia maintained, via a face mask, with 1.5% halothane in 95% O₂ / 5% CO₂ (2 L/min).

A small incision was made in the scalp to expose the skull and reveal bregma. Then, following craniotomy, a microdialysis probe, primed with modified Ringer's solution (NaCI 145 mM, KCl 4 mM, CaCl₂ 1.3 mM, pH 6.8), was implanted into the striatum (AP +1.1 mm, ML +1.5 mm, DV -3.3mm). Vital signs were monitored continually to ensure a respiratory rate of 100-190/min (average: 135 ± 20/min). Core body temperature was maintained at 38°C using a warm water-bed. Probe placement was fixed by cyanocrylate gel after which the animals were allowed to recover from the anaesthesia. Experiments were carried out the next day.

Microdialysis probes were perfused with modified Ringer's solution at a rate of 1.5 µL/min. After a washout (equilibration) period of 1.5 h, dialysis samples were collected at 20-min intervals. Drug challenges started after collection of a minimum of three consecutive basal samples confirmed a stable baseline.

### 3. Microdialysis protocols

Mice from both genotypes were randomly assigned to one of two treatment groups, destined for i.p injection of either saline or d-amphetamine (5 mg.kg i.p.). After collecting three samples, while they were in their home cage, the mice were then (individually) transferred to the dark zone of a light / dark exploration box. After 60 min (during which time baseline efflux stabilized after the handling / transfer to LDEB), animals were then injected with saline or *d*-amphetamine. After a further 30 min, they were then transferred to and confined within, the light zone of the LDEB for 1 h. Microdialysis samples were collected throughout at 20-min intervals.

At the end of each experiment, mice were killed and the brain removed. This was stored in 10% formalin at 4°C for confirmation of the correct position of the probe. It was not necessary to exclude any data on the basis of incorrect probe implantation.

### 4. HPLC and electrochemical detection

The dopamine content of the dialysis samples was determined by HPLC coupled to electrochemical detection. Solutes were separated by reversed-phase ion-pair chromatography on a Hypersil ODS 5 µm column (250 x 4.6 mm) maintained at room temperature and protected by an Aquapore guard column (30 x 4.6 mm). The mobile phase contained 0.23 mM octane sulphonic acid, 83 mM sodium dihydrogen orthophosphate, 0.84 mM EDTA, 17% methanol, adjusted to pH 4.0 and was pumped through the system at 1.0 mL/min. Dopamine was detected using a Coulochem II detector (ESA) and the mobile phase was conditioned by a guard cell (ESA model 5020). The noradrenaline content of the samples was calculated from the peak height of the chromatogram with reference to an external standard. There was no correction for probe recovery.

### 5. Drugs and reagents

d-amphetamine sulphate was obtained from Sigma-Aldrich (Poole, UK). Halothane was purchased from UCH (pharmacy). Buffer reagents were either AnalaR or HPLC grade.

### 6. Data analysis

Data from the microdialysis were analysed using repeated measures ANOVA (SPSS PC⁺). The incremental change in dopamine efflux, following injection of *d-*amphetamine was calculated by subtracting the mean efflux of the three consecutive basal samples (taken before administration of the drug and while the animal was still in the home cage) from all experimental samples. The difference in the change in noradrenaline efflux induced by *d*-amphetamine was assessed using the repeated measures two-way ANOVA. The Greenhouse - Geisser 'ε' correction was applied to correct for any violation of sphericity of the variance - covariance matrix. The criterion for statistical significance was set at *P*≤0.05.

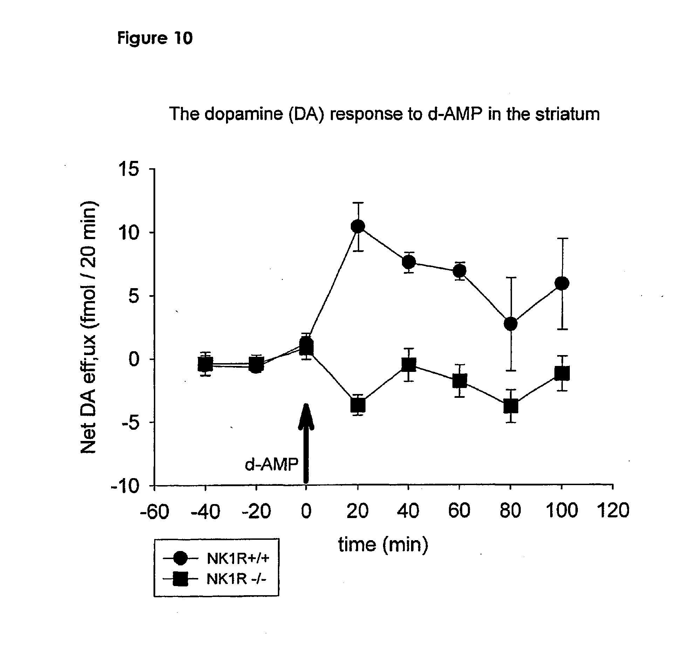

As shown in figure 10, d-AMP increased the extracellular dopamine in the frontal cortex/striatum of NK1+/+ mice but not NK1-/- mice.

### α2A-AUTORECEPTORS ON NA CELL BODIES ARE DESENSITIZED IN NK1-/- MICE Protocol

### 1. [³⁵S]GTPγS binding

The functional status of G protein-coupled α₂-adrenoceptors in NK1R+/+ and NK1R-/- mice was compared following agonist-stimulated binding of (non-hydrolysable) [³⁵S]GTPyS to α₂-adrenoceptors in the locus coeruleus and frontal cortex. Binding of [³⁵S]GTPγS to α₂-adrenoceptors was estimated essentially as described by Sim *et al.* (1995). Brain collection, storage and tissue preparation from NK1R+/+ and NK1R-/- mice was performed as described above. Coronal sections (15 µM) were brought to room temperature over 30 min. They were then incubated (15 min) in 50 mM HEPES sodium salt (pH 7.5) containing (mM) NaCl (100), MgCl₂ (3), EGTA (0.2) DTT (2). 15 min later, the buffer solution was replaced by HEPES buffer, containing GDP dilithium salt, to which the A₁ (adenosine) receptor antagonist, 8-cyclopentyl-1-3,-dipropylxanthine (DPCPX) had been added to reduce background labeling. Sections were then incubated for 3 h at 30°C in buffer containing 0.1 µM [³⁵S]GTPγS. Parallel sections included 100 µM adrenaline bitartrate to stimulate coupling of α₂ₐ-adrenoceptors to their G protein. Non-specific binding was determined by co-incubation with the α₂-adrenoceptor antagonist, RX 821002 (100 mM). The incubation was stopped by immersion in ice-cold water and, after rapid drying in cold air, sections were exposed to Biomax film (Kodak) for 2-3 days. Specific binding of [³⁵S]GTPγS was evaluated using a Microcomputer Imaging device.

### 2. Data analysis

Data from the quantitative autoradiography were analysed using the paired t-test or 1-way ANOVA with *post hoc t*-tests (with the Bonferroni correction, if appropriate).

### 3. Drugs and reagents

Adrenaline bitartrate, 8-cyclopentyl-1-3,-dipropylxanthine (DPCPX) glycylglycine HCI, guanosine 5'-O-(3-thiotriphosphate (GTPγS), guanosine 5'-diphosphate sodium (GDP), ethylene glycol-bis (2-aminoethylether-N,N,N',N'-tetraacetic acid (EGTA) were all purchased from Sigma-Aldrich (Poole, UK). Dithiothreitol was purchased from BDH (UK). RX 821002 (2-methoxy-idazoxan) hydrochloride (Tocris, Bristol, U.K.). Buffer reagents were either AnalaR or HPLC grade. GTPγS was purchased from NEN Life Sciences.

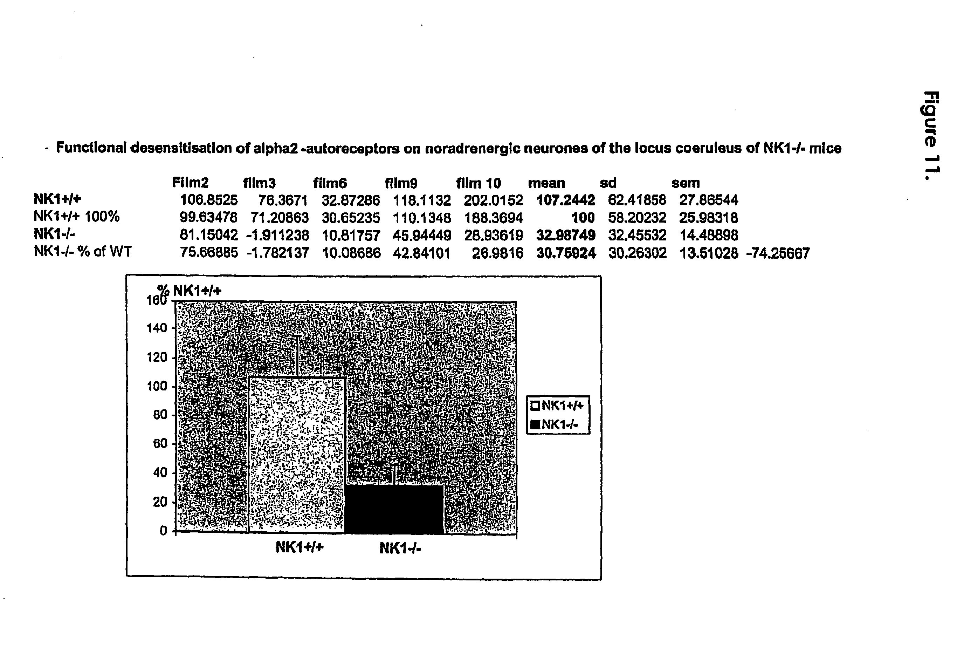

Figure 11 shows that α2A -adrenoceptors on noradrenergic neurons were desensitized in the locus coeruleus of NK1-/- mice.

### DISRUPTION OF BEHAVIOURS IN RODENTS THAT PREDICT DEPENDENCE ON D-AMPHETAMINE AND MORPHINE IN HUMANS

### Protocol

### 1. Subjects

NK1-/- mice and NK1+/+ littermates were derived from the mating of heterozygous NK1+/- mice. The targeting construct was derived from a mouse 129/sv strain genomic library and targeted clones were injected into C57BL/6 blastocysts. Chimeric males were mated with C57BL/6 females. Mice were bred from successive generations of sibling knockout and wild-type mice and can be thought of as representing a recombinent inbred strain. For CPP we also tested mice that were pure C57/B6 or 129/sv and these behave exactly as wildtype mice.

### 2. Place preference

The apparatus and conditioning procedure has been described in Wise RA & Bozarth MAA. Psychol Rec 97: 469. Analysis of CPP without food deprivation was assessed in animals with limited access to food (15% of mouse body as food with sucrose added) for 6 days before the test. Conditioning phase was performed with access to food on days 1, 3 and 5 and with no access to food on days 2, 4 and 6. Control animals had no access to food in the assigned compartments during this phase. In the food-deprived condition, mice were deprived of food the night before testing.

### 3. Aversive place conditioning

See Matthes HW et al. (1996) Loss of morphine-induced analgesia, reward effect and withdrawal symptoms in mice lacking the mu-opioid-receptor gene. Nature 383: 819. Morphine dependence was induced with twice-daily intraperitoneal administration of morphine, The doses of morphine injected at 9.00 and 19.00 on consecutive days were 1, 10 mg/kg; day 2, 20 mg/kg; day 3, 30 mg/kg, day 4, 40 mg/kg; and days 5-7, 50 mg/kg. The conditioning phase consisted of two consecutive days of alternative naloxone or vehicle injection. One hour after the morning injection of morphine, animals were injected intraperitoneally with naloxone (day 1, 1 mg/kg) or saline (day 2) and immediately confined for 20 min in the appropriate compartment.

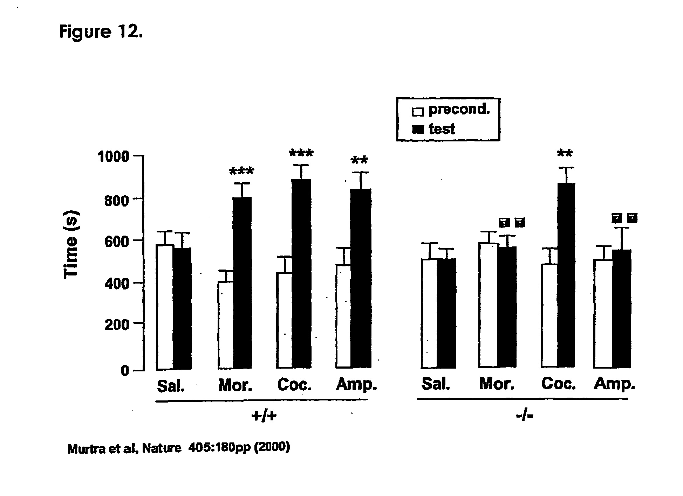

As can be seen from figure 12, the behaviours that predict dependence on d-amphetamine and morphine in humans were disrupted in NK1-/- mice. (Murtra et al., (2000) Rewarding effects of opiates are absent in mice lacking the receptor for substance P Nature. 405(6783):180-3.)

### EFFECT OF BETAXOLOL AND DESIPRAMINE ON LOCOMOTOR ACTIVITY.

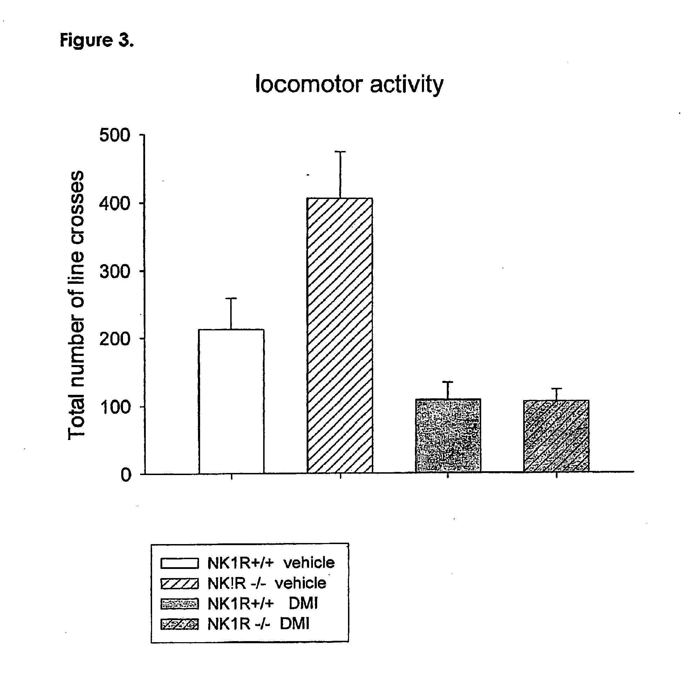

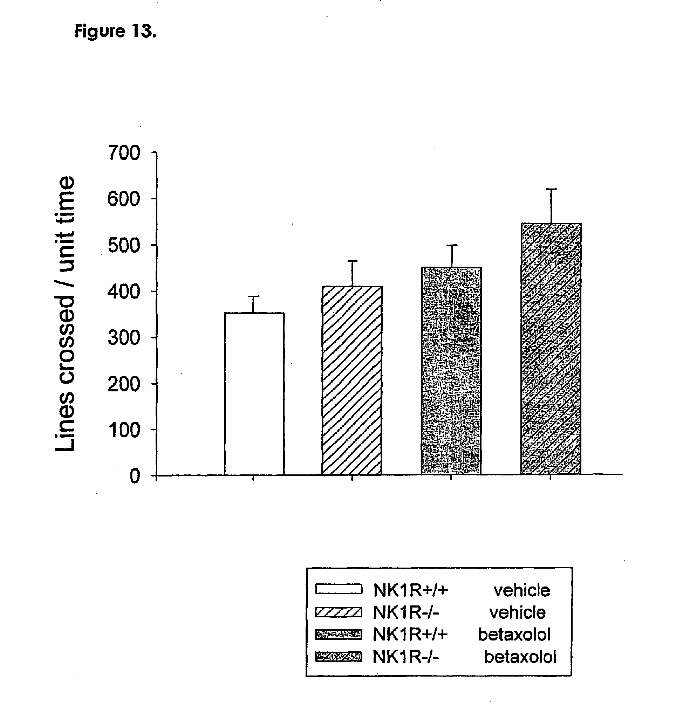

The effect of betaxolol and desipramine was tested using similar protocols to those used for testing the effect of d-amphetamine. The results are shown in figures 2, 3 and 13. The results with betaxolol (figure 13) confirm that not all drugs that increase locomotor activity in wild-type mice reduce it in NK1^{-/-} mice.

### IDENTIFICATION OF THE ROLE OF NK1 GENE IN CONDUCT DISORDER, ATTENTION DEFICIT HYPERACTIVITY DISORDER AND ALCOHOLISM

### MATERIALS AND METHODS

Linkage Disequilibrium. Linkage disequilibrium (LD) studies were performed using DNA from a population sample of neuropsychiatric disorder (conduct disorder, attention deficit hyperactivity disorder and alcoholism) patients. The population sample and LD techniques were as described as below. The present LD study took advantage of the discovery of additional physical markers identified via the physical mapping and sequencing techniques described below.

Bacterial artificial chromosome (BAC) mapping. For physical mapping, bacterial artificial chromosomes (BACs) containing human sequences were mapped to the region being analyzed based on publicly available maps (Human genome database, Toronto 1999). The BACs were then ordered and contig reconstructed by performing standard mapping with microsatellite markers and polymorphic SNP's.

### RESULTS

Alcoholism is strongly associated with childhood conduct disorder, attention deficit hyperactivity disorder as well as suicidality. It is therefore very likely that inherited personality characteristics leading to these three disorders are also increasing susceptibility to alcoholism. In order to identify genetic loci for these syndromes the large USA consortium (COGA) has been carrying out genetic linkage studies of alcoholism and comorbid disorders. This group has published evidence of linkage to several clinical phenotypes on chromosome 2p13.1. These consist of suicidality (Lod 4.2) and conduct disorder (Lod 2.2) combined with alcoholism. The positive lod scores are both very close to each other and are localised on the short arm of chromosome 2 in the 2p13.1 region. The human Tachykinin Receptor 1 (NK1) is positioned under the middle of the maximum COGA lod score peaks at 2p13.1 (Dick *et al* 2004, Hesselbrock *et al* 2004, Wiener *et al* 2005). Therefore, when viewed in the light of the behavioural characteristics of the NK1 knockout mouse, the human NK1 locus is a potential locus for increasing susceptibility to human alcoholism, attention deficit disorder, conduct disorder and suicidality. We have been able to test this hypothesis by carrying out fine mapping in a UCL based case control sample. Such a sample has the power to implicate specific genes as opposed to the COGA family linkage studies which can only implicate regions of chromosomes.

Prior to attempting to identify gene sequences, studies were performed to further narrow the neuropsychiatric disorder region. Specifically, a linkage disequilibrium (LD), allelic association and haploypic analysis was performed using population samples and techniques as described in table 5 and below.

Seven polymorphisms, which are described here for the first time, were genotyped in a sample of 600 alcoholics of UK ancestry and 600 ancestrally matched normal controls. The alcoholics also had associated attention deficit hyperactivity disorder, attention deficit disorder, criminality, conduct disorder and dis-social disorder.

None of the SNP markers we genotyped in the case control sample showed any deviation from Hardy Weinberg equilibrium supporting the accuracy of the genotyping. The results were interesting because a single nucleotide polymorphism (SNP) rs3771856 in intron 1 of NK1 showed evidence of allelic (p=0.006) and genotypic (p=0.026) association with alcoholism. These case control data were also analysed for haplotyic association with alcoholism. In this procedure markers that are very close to each other in NK1 are combined into haplotypes and estimated frequencies in cases and controls are compared. A number of two, three and four marker NK1 haplotypes as shown in table 5 were found to be significantly associated with the alcohol dependence syndrome after correction for multiple alleles and multiple markers. The empirical significances were p=0.002 for a two marker haplotype with estimated frequencies of 43% in controls and 51% in cases. Both three and four marker empirical tests of significance also showed significant haplotypic association with alcoholism (p=0.009, p=0.006 respectively). This is evidence that variation in NK1 is increasing genetic susceptibility to alcoholism and related phenotypes such as ADHD conduct disorder and suicidality. The genotyping of marker rs3771856 was validated in the UCL lab. by using a second genotyping method (TaqMan).

**Table 1**

| **SUMMARY OF CORE FEATURES OF ADHD IN HUMANS AND FINDINGS IN NK1^{-/-} MICE** | |
|---|---|
| ***ADHD in humans*** | ***Findings in NK1*^{*-*/}*⁻ mice compared to NK1*^{*+*/}*⁺ mice*** |
| Paradoxical effect of psychomotor stimulants | Locomotor activity is reduced by d-AMP and methylphenidate in NK1^{-/-} mice but increased in NK1^{+/+} mice |
| Hyperactivity, especially in an aversive (eg novel) environment | * Increased locomotor activity, especially in the novel zone of a light /dark exploration box |
| Increased impulsivity and inattentiveness, | Reduced latency to return to the novel arena from a familiar zone. Shorter, but more frequent visits to the novel test arena. |
| 'Accident-prone' / 'clumsy' | * Impaired motor co-ordination & learning in the rotarod test |
| Resembles hypofrontality (genotyping and neuroimaging suggest impaired DA transmission) | * Reduced basal extracellular DA in frontal cortex of NK1^{-/-} mice |
| | d-AMP increases extracellular DA in striatum of NK1^{+/+} mice, only |
| Evidence for increased NA transmission a_{2A}-adrenoceptor function possibly impaired (as in the Spontaneously Hypertensive rat model of ADHD] | * Increased NA release in frontal cortex |
| | * _{2A}-autoreceptors on NA cell bodies desensitized |
| Lower incidence of dependence on d-AMP in humans with ADHD | Disruption of behaviours in rodents that predict dependence on d-amphetamine and morphine in humans |

**Table 2 showing the empirical p value (1^{st} Column) from a permutation test for association between two, three and then four SNP marker haplotypes with alcoholism. The estimated haplotype frequencies are shown in cases and controls (2^{nd} and 3^{rd} columns), and in columns 4 to 10 the SNP labels are shown in row 1 with the nucleotide showing association in each haplotype shown below.**

| Empirical p value | Controls | Cases | rs3771807 | rs10210154 | rs3771827 | rs17010822 | rs12713835 | rs4853116 | rs3771856 |
|---|---|---|---|---|---|---|---|---|---|
| 0.003 | 43% | 51% | C | | | | | | G |
| 0.049 | 47% | 51% | | A | | | | | G |
| | 45% | 39% | | A | | | | | A |
| 0.016 | 39% | 46% | C | A | | | | | G |
| 0.009 | 25% | 28% | C | | C | | | | G |
| | 18% | 23% | C | | T | | | | G |
| | 2% | 4% | G | | C | | | | A |
| 0.050 | 39% | 33% | C | | | C | | | A |
| | 7% | 8% | G | | | C | | | A |
| 0.008 | 22% | 25% | C | | C | | | A | G |
| | 18% | 22% | C | | T | | | A | G |
| | 1% | 3% | G | | C | | | G | A |
| 0.006 | 28% | 32% | C | | | C | G | | G |
| | 15% | 17% | C | | | C | A | | G |
| | 22% | 23% | C | | | C | A | | A |

## Claims

1. The use of a NK1^{-/-} rodent or a rodent having a functional substance P receptor to which an antagonist of the substance P receptor has been administered as an animal model for ADHD, wherein the rodent shows reduced locomotor activity in response to methylphenidate or d-amphetamine.

2. A method for identifying a compound that affects ADHD, comprising administering a test compound to a rodent according to claim 1, wherein the compound affects ADHD if administration of the compound results in a change in the rodent's locomotor activity in response to methylphenidate or d-amphetamine.

3. A wild-type NK1 gene or an NK1 agonist or an ACE inhibitor for use in the treatment of ADHD.

4. A vector comprising a wild-type NK1 gene for use in the treatment of ADHD.

## Patentansprüche

1. Verwendung eines NK1^{-/-}-Nagers oder eines Nagers mit einem funktionellen Substanz P-Rezeptor, dem ein Antagonist für den Substanz P-Rezeptor verabreicht wurde, als Tiermodell für ADHD, wobei der Nager als Reaktion auf Methylphenidat oder d-Amphetamin verminderte Bewegungsaktivität zeigt.

2. Verfahren zum Identifizieren einer Verbindung, die ADHD beeinflusst, umfassend Verabreichen einer Testverbindung an einen Nager nach Anspruch 1, wobei die Verbindung ADHD beeinflusst, wenn die Verabreichung der Verbindung zu einer Veränderung der Bewegungsaktivität des Nagers als Reaktion auf Methylphenidat oder d-Amphetamin führt.

3. Wildtyp-NK1-Gen oder NK1-Antagonist oder ACE-Inhibitor zur Verwendung bei der Behandlung von ADHD.

4. Vektor umfassend ein Wildtyp-NK1-Gen zur Verwendung bei der Behandlung von ADHD.

## Revendications

1. Utilisation d'un rongeur NK1^{-/-} ou d'un rongeur possédant un récepteur fonctionnel de la substance P, auquel un antagoniste du récepteur de la substance P a été administré, en tant que modèle animal de l'ADHD, où le rongeur montre une activité locomotrice réduite en réponse au méthylphénidate ou à la d-amphétamine.

2. Procédé pour identifier un composé qui affecte l'ADHD, comprenant l'administration d'un composé test à un rongeur selon la revendication 1, dans lequel le composé affecte l'ADHD si l'administration du composé entraîne une modification de l'activité locomotrice du rongeur en réponse au méthylphénidate ou à la d-amphétamine.

3. Gène NK1 de type sauvage ou agoniste de NK1 ou inhibiteur de l'ECA destiné à être utilisé dans le traitement de l'ADHD.

4. Vecteur comprenant un gène NK1 de type sauvage destiné à être utilisé dans le traitement de l'ADHD.
